# EUROPÄISCHE PATENTSCHRIFT

(11) **EP 1 368 302 B1**
(45) Veröffentlichungstag und Bekanntmachung des Hinweises auf die Patenterteilung: **10.09.2008**
(21) Anmeldenummer: 02718141.1
(22) Anmeldetag: 18.02.2002
(51) Int. Cl.: C07C 229/38, A61K 31/195, A61P 9/00

(54) **NEUARTIGE AMINODICARBONSÄUREDERIVATE**
NOVEL AMINO DICARBOXYLIC ACID DERIVATIVES
NOUVEAUX DERIVES D'ACIDE AMINODICARBOXYLIQUE

(30) Priorität: 01.03.2001 DE 10109859
(43) Veröffentlichungstag der Anmeldung: 10.12.2003
(73) Patentinhaber: Bayer HealthCare AG, 51368 Leverkusen (DE)
(72) Erfinder: HÄRTER, Michael, 51375 Leverkusen (DE); HAHN, Michael, 40764 Langenfeld (DE); HIRTH-DIETRICH, Claudia, 42115 Wuppertal (DE); KNORR, Andreas, 40699 Erkrath (DE); STAHL, Elke, 51467 Bergisch Gladbach (DE); STASCH, Johannes-Peter, 42651 Solingen (DE); WUNDER, Frank, D-42117 Wuppertal (DE)
(86) Internationale Anmeldenummer: PCT/EP2002/001682
(87) Internationale Veröffentlichungsnummer: WO 2002/070459

(56) Entgegenhaltungen:
- WO-A-01/19780
- WO-A-98/16223
- US-A- 5 674 909

## Beschreibung

Die vorliegende Erfindung betrifft neue Aminocarbonsäurederivate, welche die lösliche Guanylatcyclase auch über einen neuartigen, ohne Beteiligung der Häm-Gruppe des Enzyms verlaufenden Wirkmechanismus stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587), sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung (Gerzer et al., FEBS Lett. 132(1981), 71), oder über einen Häm-abhängigen Mechanismus, der unabhängig von NO ist, aber zu einer Potenzierung der stimulierenden Wirkung von NO oder CO führt (z.B. YC-1, Hoenicka et al., J. Mol. Med. (1999) 14; oder die in der WO 98/16223, WO 98/16507 und WO 98/23619 beschriebenen Pyrazolderivate).

Die in der Literatur behauptete stimulierende Wirkung von Isoliquiritigenin und von Fettsäuren, wie z.B. Arachidonsäure, Prostaglandinendoperoxide und Fettsäurehydroperoxide auf die lösliche Guanylatcyclase konnte nicht bestätigt werden (vgl. z.B. Hoenicka et al., J. Mol. Med. 77 (1999), 14).

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben (Ignarro et al., Adv. Pharmacol. 26 (1994), 35). Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird (Mülsch et al., Naunyn Schmiedebergs Arch. Pharmacol. 355, R47 ).

Im Gegensatz zu den vorstehend beschriebenen, aus dem Stand der Technik als Stimulatoren der löslichen Guanylatcyclase bekannten Verbindungen sind die erfindungsgemäßen Verbindungen in der Lage, sowohl die Häm-haltige als auch die Häm-freie Form der löslichen Guanylatcyclase zu stimulieren. Die Stimulierung des Enzyms verläuft bei diesen neuen Stimulatoren also über einen Häm-unabhängigen Weg, was auch dadurch belegt wird, dass die neuen Stimulatoren am Häm-haltigen Enzym einerseits keine synergistische Wirkung mit NO zeigen und andererseits sich die Wirkung dieser neuartigen Stimulatoren nicht durch den Häm-abhängigen Inhibitor der löslichen Guanylatcyclase, 1*H*-1,2,4-Oxadiazol-(4,3-a)-chinoxalin-1-on (ODQ), blockieren lässt.

Dies stellt einen neuen Therapieansatz zur Behandlung von Herz-Kreislauferkrankungen und anderen über eine Beeinflussung des cGMP-Signalweges in Organismen therapierbaren Erkrankungen dar.

In der EP-A-0 345 068 ist unter anderem die Aminoalkancarbonsäure (1) als Zwischenprodukt bei der Synthese von GABA-Antagonisten beschrieben:

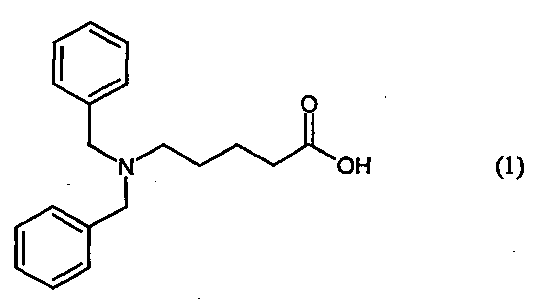

In der WO 93/00359 ist die Aminoalkancarbonsäure (2) als Intermediat in der Peptid-Synthese sowie dessen Verwendung als Wirkstoff zur Behandlung von Erkrankungen des zentralen Nervensystems beschrieben:

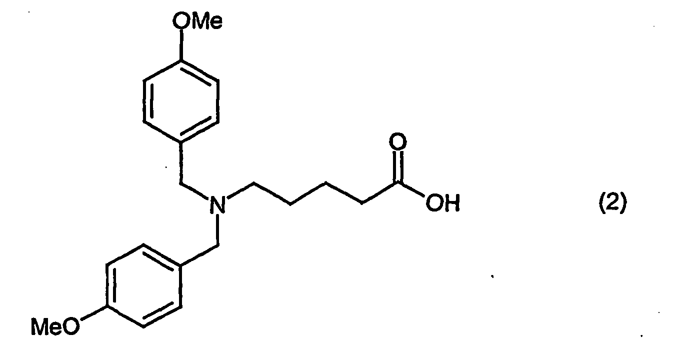

In keiner dieser beiden Schriften ist jedoch beschrieben, dass derartige Aminoalkancarbonsäuren einen von der im Enzym befindlichen Häm-Gruppe unabhängigen stimulierenden Effekt auf die lösliche Guanylatcyclase ausüben können.

Den erfindungsgemäßen Verbindungen strukturell ähnliche Substanzen sind darüber hinaus aus WO 01/19776, WO 01/19355, WO 01/19780 und WO 01/19778 bekannt.

Die vorliegende Erfindung betrifft Verbindungen der allgemeinen Formel (I) 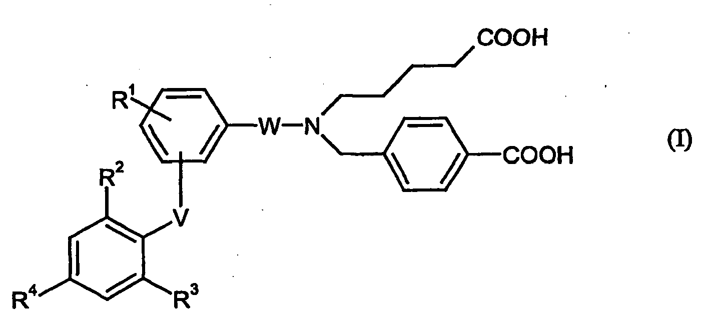 worin
- R¹: in meta- oder para-Position zum Rest W angeordnet ist und einen Rest aus der Gruppe, bestehend aus H, Halogen oder OCF₃, bedeutet;
- R²: H oder Halogen bedeutet;
- R³: H oder Halogen bedeutet;
- R⁴: C₁₋₆-Alkyl, C₃₋₈-Cycloalkyl, CF₃, OCF₃, F, Cl, CN, OMe oder Phenyl bedeutet, wobei der Phenylrest zusätzlich einen Substituenten aus der Gruppe, bestehend aus Halogen, CN, C₁₋₆-Alkoxy, CF₃, C₁₋₆-Alkyl, tragen kann;
- V: in ortho- oder meta-Position zum Rest W angeordnet ist und für O, CH₂O, OCF₂, oder O-C₁₋₆-Alkyl-O steht;
- W: für CH₂ oder CH₂CH₂ steht;
sowie Salze, Isomere und Hydrate davon.

Gemäß einer bevorzugten Ausführungsform betrifft die vorliegende Erfindung Verbindungen der Formel (I), worin
- R¹: in meta-Position zum Rest W angeordnet ist und einen Rest aus der Gruppe, bestehend aus H oder Halogen, bedeutet;
- R²: H oder Halogen bedeutet;
- R³: H oder Halogen bedeutet;
- R⁴: C₁₋₆-Alkyl, C₃₋₈-Cycloalkyl oder Phenyl bedeutet, wobei der Phenylrest zusätzlich einen Substituenten aus der Gruppe, bestehend aus Halogen, CN, C₁₋₆-Alkoxy, CF₃, C₁₋₆-Alkyl, tragen kann;
- V: in ortho- oder meta-Position zum Rest W angeordnet ist und für O, CH₂O, OCF₂ oder O-C₁₋₆-Alkyl-O steht;
- W: für CH₂ oder CH₂CH₂ steht;
sowie Salze, Isomere und Hydrate davon.

Gemäß einer besonders bevorzugten Ausführungsform betrifft die vorliegende Erfindung Verbindungen der Formel (I), worin
- R¹: in meta-Position zum Rest W angeordnet ist und einen Rest aus der Gruppe, bestehend aus H, F, Cl oder Br, bedeutet;
- R2: H bedeutet;
- R3: H bedeutet;
- R⁴: Methyl, Ethyl, n-Propyl, i-Propyl, n-Butyl, i-Butyl, t-Butyl, Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CN, Methoxy, Ethoxy, n-Propoxy, i-Propoxy, n-Butyloxy, i-Butyloxy, t-Butyloxy, CF₃, Methyl, Ethyl, n-Propyl, i-Propyl, n-Butyl, i-Butyl, t-Butyl, tragen kann;
- V: in ortho- oder meta-Position zum Rest W angeordnet ist und für O, CH₂O, OCF₂ oder O-C₁₋₆-Alkyl-O steht;
- W: für CH₂ oder CH₂CH₂ steht;
sowie Salze, Isomere und Hydrate davon.

Gemäß einer weiteren besonders bevorzugten Ausführungsform betrifft die vorliegende Erfindung Verbindungen der Formel (I), worin
- R¹: in meta-Position zum Rest W angeordnet ist und H bedeutet;
- R2: H bedeutet;
- R3: H bedeutet;
- R⁴: Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CF₃, tragen kann;
- V: in meta-Position zum Rest W angeordnet ist und für O steht;
- W: für CH₂ steht;
sowie Salze, Isomere und Hydrate davon.

Gemäß einer weiteren besonders bevorzugten Ausführungsform betrifft die vorliegende Erfindung Verbindungen der Formel (I), worin
- R¹: in meta-Position zum Rest W angeordnet ist und H bedeutet;
- R²: H bedeutet;
- R³: H bedeutet;
- R⁴: Phenyl bedeutet, wobei der Phenylrest zusätzlich einen Substituenten aus der Gruppe, bestehend aus F, Cl, Br, OMe, CF₃, Methyl, Ethyl, n-Propyl, i-Propyl, n-Butyl, i-Butyl, t-Butyl, tragen kann;
- V: in ortho-Position zum Rest W angeordnet ist und für OCF₂ steht;
- W: für CH₂CH₂ steht;
sowie Salze, Isomere und Hydrate davon.

Gemäß einer weiteren besonders bevorzugten Ausführungsform betrifft die vorliegende Erfindung Verbindungen der Formel (I), worin
- R¹: in meta-Position zum Rest W angeordnet ist und einen Rest aus der Gruppe, bestehend aus H, F, Cl oder Br, bedeutet;
- R²: H bedeutet;
- R³: H bedeutet;
- R⁴: Methyl, Ethyl, n-Propyl, i-Propyl, n-Butyl, i-Butyl, t-Butyl, Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CN, OMe, CF₃, Methyl, Ethyl, n-Propyl, i-Propyl, n-Butyl, i-Butyl, t-Butyl, tragen kann;
- V: in ortho-Position zum Rest W angeordnet ist und für CH₂O steht;
- W: für CH₂CH₂ steht;
sowie Salze, Isomere und Hydrate davon.

Die erfindungsgemäßen Verbindungen der allgemeinen Formel (I) können auch in Form ihrer Salze vorliegen. Im Allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch Racematspaltung oder chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Im Rahmen der vorliegenden Erfindung haben die Substituenten soweit nicht anders angegeben im Allgemeinen die folgende Bedeutung:
Alkyl steht im Allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkoxy steht im Allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Cycloalkyl steht im Allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Halogen steht im Rahmen der Erfindung für Fluor, Chlor, Brom und Iod.

Im Rahmen der vorliegenden Erfindung sind die Definitionen für den Rest V so zu verstehen, dass das zuerst genannte Atom an den Phenylring gebunden ist, der auch den Rest R¹ trägt. Im Fall von V = OCF₂ ist also das Sauerstoffatom an den Phenylring gebunden ist, der auch den Rest R¹ trägt.

Die vorliegende Erfindung betrifft weiterhin ein Verfahren zur Herstellung der Verbindungen der Formel (I), dadurch gekennzeichnet, dass man

Verbindungen der Formel (II) 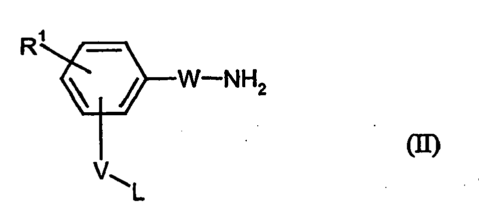 worin
- R¹, V: und W die in Anspruch 1 angegebenen Bedeutungen haben, und
- L: für den Fall, dass V gleich O ist, für Methyl oder ansonsten für einen Rest der Formel 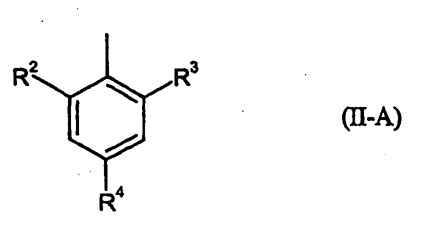 steht, wobei R², R³ und R⁴ die vorstehend angegebenen Bedeutungen haben,
mit einem 4-Formylbenzoesäure-C₁₋₆-alkylester in einem organischen Lösungsmittel gegebenenfalls unter Erhitzen und gleichzeitiger oder anschließender Zugabe eines Reduktionsmittels zu Verbindungen der Formel (III) umsetzt, 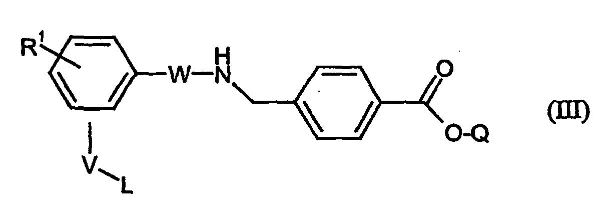 worin R¹, V, W und L die vorstehend angegebenen Bedeutungen haben und Q für einen C₁₋₆-Akylrest steht,
anschließend - gegebenenfalls unter vorheriger Etherspaltung zur freien Hydroxylgruppe, wenn V für O und L für Methyl steht - mit einem ω-Halogenvaleriansäure-C₁₋₆-alkylester in einem organischen Lösungsmittel in Gegenwart einer Base unter Erhitzen zu Verbindungen der Formel (IV) umsetzt, 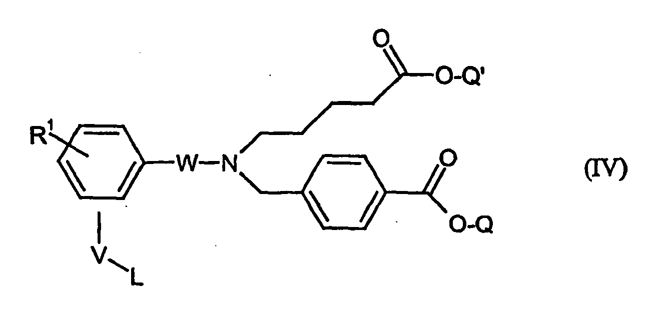 worin R¹, V,W und Q die vorstehend angegebenen Bedeutungen haben, Q' für einen C₁₋₆-Alkylrest steht und L für H - wenn V gleich O ist - oder einen Rest der Formel II-A steht,
anschließend - für den Fall, dass V gleich O und L für H steht - mit einer Verbindung der Formel IV-A in einem organischen Lösungsmittel unter Erhitzen 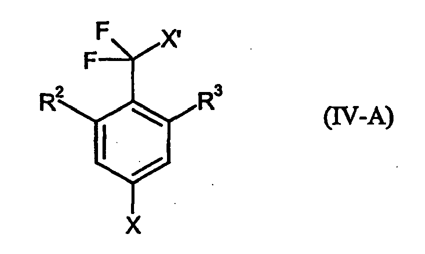 wobei R² und R³ die in Anspruch 1 angegebene Bedeutung haben und X und X' jeweils für Halogen stehen,
und anschließender Palladium-katalysierter Substitution des Restes X mit einem Benzolboronsäurederivat zu Verbindungen der Formel (V) umsetzt, 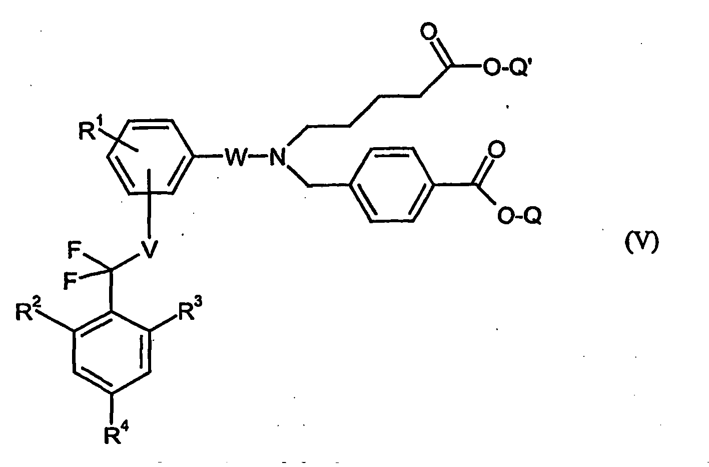 und anschließend die Verbindungen der Formel (IV) beziehungsweise (V) unter alkalischen Bedingungen zu den Verbindungen der Formel (I) hydrolysiert.

Die für die erfindungsgemäßen Verfahren bevorzugten Basen umfassen herkömmlicherweise für basische Reaktionen eingesetzte basische Verbindungen. Vorzugsweise können Alkalimetallhydride wie beispielsweise Natriumhydrid oder Kaliumhydrid, oder Alkalimetallalkoholate wie Natriummethanolat, Natriumethanolat, Kaliummethanolat, Kaliumethanolat oder Kalium-t.-butylat, oder Carbonate wie Natriumcarbonat, Cäsiumcarbonat oder Kaliumcarbonat oder Amide wie Natriumamid oder Lithiumdiisopropylamid, oder Organolithium-Verbindungen wie Phenyllithium, Butyllithium oder Methyllithium oder Natriumhexamethyldisilazan verwendet werden.

Für die Umsetzung der Verbindungen der Formel (II) zu den Verbindungen der Formel (III) bevorzugte Lösungsmittel sind herkömmliche organische Lösungsmittel, welche sich unter den Reaktionsbedingungen nicht verändern. Vorzugsweise können für das erfindungsgemäße Verfahren Ether wie Diethylether, Butylmethylether, Dioxan, Tetrahydrofuran, Glykoldimethylether oder Diethylenglykoldimethylether, oder Kohlenwasserstoffe wie Benzol, Toluol, Xylol oder Petrolether, oder Alkohole wie Methanol oder Ethanol oder halogenierte Kohlenwasserstoffe wie Tetrachlorkohlenstoff, Chlormethan oder Dichlormethan verwendet werden. Es ist selbstverständlich auch möglich, Gemische der vorstehend genannten Lösungsmittel zu verwenden. Erfindungsgemäß bevorzugt ist die Verwendung von Ethanol, Methanol, Dichlormethan oder Toluol.

Die Verbindungen der Formel (II) werden zunächst mit einem 4-Formylbenzoesäure-C₁₋₆-alkylester zu einer Schiffschen Base umgesetzt und diese dann mit gängigen Reduktionsmitteln, wie z.B. NaBH₄, H₂/Pd/C usw. reduziert oder direkt unter den Bedingungen einer reduktiven Alkylierung in Gegenwart eines Reduktionsmittels, wie z.B. H₂/Pd/C, NaCNBH₃, NaH(OAc)₃ umgesetzt (vgl. Patai, Ed., The Chemistry of the Carbon-Nitrogen Double Bond, S. 276-293 und die dort zitierte Literatur). Je nach Beschaffenheit der Ausgangsverbindung kann hierbei die Umsetzung bei Raumtemperatur erfolgen oder muss auf 50 bis 110°C für mehrere Stunden bis mehrere Tage erhitzt werden. Die Reaktion kann bei Normaldruck, erhöhtem oder verringertem Druck ausgeführt werden (beispielsweise in einem Bereich von 0,5 bis 5 bar). Im Allgemeinen wird die Reaktion bei Normaldruck ausgeführt. 4-Formylbenzoesäure-C₁₋₆-alkylester sind kommerziell erhältlich, literaturbekannt, oder können in Analogie zu literaturbekannten Verfahren synthetisiert werden (vgl. z.B. J. Med. Chem. 1989, 32, 1277; Chem. Ber. 1938, 71, 335; Bull. Soc. Chim. Fr. 1996, 123, 679, WO96/11902; DE-2209128; Synthesis 1995, 1135; Bull. Chem. Soc. Jpn. 1985, 58, 2192, Synthesis 1983, 942; J. Am. Chem. Soc. 1992, 114, 8158).

Die Umsetzung der Verbindungen der Formel (III) zu den Verbindungen der Formel (IV) kann vorzugsweise in Acetonitril oder Butyronitril jeweils durch Reaktion der Verbindungen (II) und (III), (IV) und (V) beziehungsweise (VI) und (VII) in Gegenwart einer Base wie Natriumcarbonat, Et₃N, DABCO, K₂CO₃, KOH, NaOH oder NaH durchgeführt werden. Die Reaktion kann im Allgemeinen in einem Temperaturbereich von -20°C bis +90°C, vorzugsweise von 0°C bis +70°C ausgeführt werden. Die Reaktion kann bei Normaldruck, erhöhtem oder verringertem Druck ausgeführt werden (beispielsweise in einem Bereich von 0,5 bis 5 bar). Im Allgemeinen wird die Reaktion bei Normaldruck ausgeführt. Als Lösungsmittel kommen jedoch prinzipiell die vorstehend für die Umsetzung der Verbindungen der Formel (II) zu den Verbindungen der Formel (III) genannten Lösungsmittel in Frage. Als ω-Halogenvaleriansäurealkylester wird erfindungsgemäß bevorzugt der entsprechende ω-Bromvaleriansäuremethylester verwendet. ω-Halogenvaleriansäurealkylester sind kommerziell erhältlich, literaturbekannt oder können nach literaturbekannten Verfahren synthetisiert werden (vgl. z.B. J. Chem. Soc. 1958,3065).

Für den Fall, dass V gleich O und L gleich Methyl ist, sollte vor der Umsetzung der entsprechenden Verbindung der Formel (III) mit dem ω-Halogenvaleriansäurealkylester die vorhandene Methoxygruppe in die freie Hydroxygruppe überführt werden. Dies kann auf bekannte Art erfolgen (vgl. hierzu T.W. Greene, P.G.M. Wuts, Protective Groups in Organic Synthesis, second edition, New York, 1991). Beispielsweise lässt sich die Methylgruppe unter Bildung des Phenols durch Bortribromid in Methylenchlorid bei -70 bis 20°C, durch Trimethylsilyliodid in Chloroform bei 25-50°C oder durch Natriumethylthiolat in DMF bei 150°C abspalten. Erfindungsgemäß ist die Umsetzung mit Bortribromid bevorzugt.

Die Verbindungen der Formel (IV) werden anschließend beispielsweise durch Zugabe wässriger Lösungen starker Säuren wie z.B. HCl oder H₂SO₄, oder starker Basen wie z.B. NaOH, KOH oder LiOH in die Verbindungen der Formel (I) durch Hydrolyse der Esterfunktionen zu den freien Carboxylgruppen überführt. Die Reaktion kann in einem der vorstehend genannten organischen Lösungsmitteln, in Wasser oder in Gemischen aus organischen Lösungsmitteln oder in Gemischen aus organischen Lösungsmitteln mit Wasser durchgeführt werden. Erfindungsgemäß bevorzugt ist beispielsweise die Durchführung der Reaktion in einem Gemisch aus Wasser und Methanol oder Dioxan. Die Reaktion kann im Allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im Allgemeinen wird die Reaktion bei Normaldruck ausgeführt.

Für den Fall, dass bei den Verbindungen der Formel (IV) V gleich O und L gleich H ist, wird vor der vorstehend beschriebenen Hydrolyse der Esterfunktionen zunächst eine Umsetzung mit den Verbindungen der Formel (IV-A) durchgeführt. Hierbei handelt es sich um eine nukleophile Substitution einer Abgangsgruppe X' in der Verbindung der Formel (IV-A) durch die Hydroxyfunktion der Verbindung der Formel (IV) dargestellt. Als Abgangsgruppe X' kommen hierbei beispielsweise in Frage: Halogen, Tosylat, Mesylat, oder eine durch Reagenzien wie Diisopropylazo-dicarboxylat/PPh₃ aktivierte Hydroxyfunktion (Mitsonobu-Reaktion). Bevorzugt ist X' gleich Halogen, besonders bevorzugt gleich Br. Diese Reaktion kann vorzugsweise in Diemthylformamid (DMF) durch Reaktion der Verbindungen (IV) und (IV-A) in Gegenwart einer Base wie Natriumcarbonat, Kaliumcarbonat, Et₃N, DABCO, K₂CO₃, KOH, NaOH oder vorzugsweise NaH durchgeführt werden. Die Reaktion kann im Allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im Allgemeinen wird die Reaktion bei Normaldruck ausgeführt.

Die Verbindungen der Formel (IV-A) können aus kommerziell erhältlichen, literaturbekannten oder in Analogie zu literaturbekannten Verfahren synthetisierbaren Verbindungen (vgl. z.B. J. prakt. Chem. 1960, 341; Farmaco Ed. Sci. 1956, 378; Eur. J. Med. Chem. Chim. Ther. 1984, 19, 205; Bull. Soc. Chim. Fr. 1951, 97. Liebigs Ann. Chem. 1954, 586, 52; EP-A-0 334 137) durch NBS-Bromierung der Difluormethylgruppe in Analogie zu literaturbekannten Verfahren (vgl. z.B. Monatsh. Chem. 1996, 127(2), 201-217; J. Med. Chem. 1992, 35, 368; J. heterocycl. Chem. 24 (1987), N3, 725-731; Synth. Commun. 1996, 26(15), 2803-2809) erhalten werden.

Anschließend wird die so erhaltene Verbindung , welche eine substituierbare Gruppe X enthält, durch Umsetzung mit einem Benzolboronsäurederivat in Gegenwart einer Palladiumverbindung sowie gegebenenfalls eines Reduktionsmittels und weiterer Zusatzstoffe im basischen Medium dargestellt. Die Reaktion stellt formal eine reduktive Kupplung dar, wie sie z.B. in L.S. Hegedus, Organometallics in Synthesis, M. Schlosser, Ed., Wiley & Sons, 1994, beschrieben ist. Als substituierbare Gruppe X kann beispielsweise ein Halogenrest wie Br oder I oder eine herkömmliche Abgangsgruppe wie beispielsweise ein Triflatrest verwendet werden. Erfindungsgemäß bevorzugt ist ein Halogenrest, insbesondere Br. Als Palladiumverbindung kann eine Palladium(II)-Verbindung wie z.B. Cl₂Pd(PPh₃)₂ oder Pd(OAc)₂ oder eine Palladium(0)-Verbindung wie z.B. Pd(PPh₃)₄ oder Pd₂(dba)₃ verwendet werden. Falls erforderlich, können dem Reaktionsgemisch noch zusätzlich ein Reduktionsmittel wie beispielsweise Triphenylphosphin oder andere Zusatzstoffe wie beispielsweise Cu(I)Br, NBu₄NCl, LiCl oder Ag₃PO₄ zugesetzt werden (vgl. hierzu T Jeffery, Tetrahedron lett. 1985, 26, 2667-2670; T. Jeffery, J. Chem. Soc., Chem. Commun. 1984, 1287-1289; S. Bräse, A. deMejiere in "Metal-catalyzied cross-coupling reactions", Ed. F. Diederich, P. J. Stang, Wiley-VCH, Weinheim 1998, 99-166). Die Reaktion wird in Gegenwart einer herkömmlichen Base wie z.B. Na₂CO₃, NaOH oder Triethylamin durchgeführt. Als Lösungsmittel kommen die vorstehend genannten organischen Lösungsmittel in Frage, wobei Ether wie beispielsweise 1,2-Dimethoxyethan besonders bevorzugt sind. Die Reaktion kann im Allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im Allgemeinen wird die Reaktion bei Normaldruck ausgeführt.

Benzolboronsäuren sind kommerziell erhältlich, literaturbekannt, oder können in Analogie zu literaturbekannten Verfahren synthetisiert werden (vgl. z.B. J.Chem.Soc.C 1966,566. J.Org.Chem., 38, 1973, 4016).

Die so dargestellten Verbindungen der Formel (V) können anschließend wie vorstehend beschrieben zu den Verbindungen der Formel (I) hydrolysiert werden.

Die Verbindungen der Formel (II) sind auf unterschiedliche Weise zugänglich, sofern sie nicht kommerziell erhältlich sind. So können Verbindungen der Formel (II), bei denen V gleich CH₂O ist, durch Veresterung käuflich erhältlicher Methylbenzoesäuren, NBS-Bromierung der Methylgruppe in Analogie zu literaturbekannten Verfahren (vgl. z.B. Monatsh. Chem. 1996, 127(2), 201-217; J. Med. Chem. 1992, 35, 368; J. heterocycl. Chem. 24 (1987), N3, 725-731; Synth. Commun. 1996, 26(15), 2803-2809) zu den entsprechenden Brommethylverbindungen, anschließende Substitution der eingeführten Bromgruppe durch die phenolische OH-Gruppe einer käuflich erhältlichen Phenolverbindung in einem organischen Lösungsmittel wie Acetonitril in Gegenwart einer Base wie beispielsweise Kaliumcarbonat, sowie Überführung der Estergruppe durch Reduktion mit gängigen Reduktionsmitteln wie beispielsweise LiAlH₄, Überführung in das entsprechende Benzylhalogenid mit beispielsweise SOCl₂ oder mit Tetrabrommethan/Triphenylphosphin in einem organischen Lösungsmittel wie Diethylether, nukleophile Substitution mit einem Cyanid wie beispielsweise NaCN oder Trimethylsilylcyanid und Reduktion der Nitrilfunktion durch literaturbekannte Methoden wie beispielsweise BH₃·THF, BH₃·S(CH₃)₂ oder LiAlH₄/AlCl₃ erhalten werden.

Für den Fall, dass V gleich O ist, kann man ausgehend von käuflich erhältlichen. literaturbekannten oder in Analogie zu literaturbekannten Verfahren synthetisierbaren Halogenbenzonitrilen (vgl. z.B. Chem. Pharm. Bull. 31, 10, 1983, 3424-3445; Bull. Chem. Soc. Fr. <II>, 1979, 241-248; Chem. Ber. 80, 1947, 469-472, J. Chem. Soc. 1933, 489-493) die Verbindungen der Formel (II) durch Umsetzung mit einer käuflich erhältlichen Phenolverbindung in einem organischen Lösungsmittel wie Pyridin in Gegenwart einer Base wie beispielsweise Kaliumcarbonat und in Gegenwart von CuI unter Schutzgasatmosphäre und Erhitzen, sowie anschließende Reduktion der Nitrilfunktion wie vorstehend beschrieben darstellen.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen der allgemeinen Formel (I), führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, fibrotischen Erkrankungen wie Leberfibrose oder Lungenfibrose, asthmatischen Erkrankungen und Krankheiten des Urogenitalsystems wie beispielsweise Prostatahypertrophie, erektile Dysfunktion, weibliche sexuelle Dysfunktion und Inkontinenz sowie zur Behandlung von Glaucoma eingesetzt werden.

Die in der vorliegenden Erfindung beschriebenen Verbindungen der allgemeinen Formel (I), stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen der allgemeinen Formel (I) zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Als besonderes und überraschendes Merkmal weisen die Verbindungen der vorliegenden Erfindung eine unerwartet lange Wirkdauer auf.

### Gefäßrelaxierende Wirkung in vitro

Kaninchen werden durch intravenöse Injektion von Thiopental-Natrium narkotisiert bzw. getötet (ca. 50 mg/kg) und entblutet Die Arteria Saphena wird entnommen und in 3 mm breite Ringe geteilt. Die Ringe werden einzeln auf je einem triangelförmigen, am Ende offenen Häkchenpaar aus 0,3 mm starkem Spezialdraht (Remanium^{®}) montiert. Jeder Ring wird unter Vorspannung in 5 ml Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O: 1,4; KH₂PO₄: 1,2; NaHCO₃: 25; Glucose: 10; Rinderserumalbumin: 0,001 %. Die Kontraktionskraft wird mit Statham UC2-Zellen erfasst, verstärkt und über A/D-Wandler (DAS-1802 HC, Keithley Instruments München) digitalisiert, sowie parallel auf Linienschreibem registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50 % zu reduzieren (IC₅₀). Das Standardapplikationsvolumen beträgt 5 µl. Der DMSO-Anteil in der Badlösung entspricht 0,1 %.

Die Ergebnisse sind in Tabelle 1 gezeigt:

**Tabelle 1: Gefäßrelaxierende Wirkung in vitro**

| **Beispiel** | **IC₅₀ (nM)** |
|---|---|
| 2 | 55 |
| 3 | 36 |
| 6 | 0,041 |
| 12 | 0,4 |
| 17 | 0,26 |

### Stimulation der rekombinanten löslichen Guanylatcyclase (sGC) in vitro

Die Untersuchungen zur Stimulation der rekombinanten löslichen Guanylatcyclase (sGC) und die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77 (1999): 14-23.

Die Häm-freie Guanylatcyclase wurde durch Zugabe von Tween 20 zum Probenpuffer (0,5% in der Endkonzentration) erhalten.

Die Aktivierung der sGC durch eine Prüfsubstanz wird als n-fache Stimulation der Basalaktivität angegeben.

### Untersuchung der antifibrotischen Substanzwirkung in-vivo

### Methodik

Die antifibrotische Wirkung der Substanzen wurde im Modell der Schweineseruminduzierten Leberfibrose bei der Ratte untersucht. Die Behandlung mit heterologem Serum, z.B. Schweineserum bei Ratten, ist eine häufig in der Literatur angewendete Methode zur Auslösung einer Leberfibrose mit anschließender Zirrhose, die im Gegensatz zu anderen Modellen nur eine minimale Schädigung und Entzündung der Leberzparenchymzellen hervorruft (Bhunchet, E. and Wake, K. (1992): Role of mesenchymal cell populations in porcine serum-induced rat liver fibrosis. Hepatology 16: 1452-1473). Weibliche Sprague Dawley Ratten wurden 2 x wöchentlich mit 0.5 ml/Tier sterilem Schweineserum (Sigma) i.p. behandelt, Kontrolltiere mit steriler physiologischer Kochsalzlösung (2 x wöchentlich 0.5 ml/Tier i.p.).Die Behandlung mit Prüfsubstanz (1 x täglich in 5 ml/kg p.o. Lösungsmittel bestehend aus 20% Cremophor, 10% Transcutol and 70 % H₂O) erfolgte parallel zur Schweineserum-Behandlung. Nach sieben Wochen Behandlung wurden die Tiere getötet und die Lebern zur Quantifizierung des Kollagengehalts entnommen.

Für die histologische Untersuchung des Lebergewebes wurden standardisierte transverse Gewebezylinder (etwa 10 x 2 mm) aus dem rechten Vorderlappen der Leber gestanzt. Gefrierschnitte wurden für die Detektion von durch Leberfibrose hervorgerufenem Narbenkollagen mit 0,1 %iger Pikrosiriusrot-Lösung gefärbt.

Fast Green wurde als Gegenfärbung zur Kontrastverstärkung eingesetzt. Das Ausmaß der Leberfibrose wurde in jedem Schnitt als prozentualer Anteil der Pikrosiriusrotgefärbten Fläche an der gesamten Meßfläche bestimmt. Die Parameter der videomikroskopischen Farbdetektion wurden standardisiert und im gesamten Experiment konstant gehalten. 64 Felder eines standardisierten Rasters von 31 mm² wurden bei 100-facher Endvergrößerung ausgemessen. Für die halbautomatische Morphometrie wurde ein Leica Quantimed 500MC (Leica Deutschland) eingesetzt.

Zur OH-Prolin-Bestimmung nach Prockop und Udenfried (Prockop, D.J. and Udenfried, S.A. (1960): A specific method for the analysis of hydroxyproline in tissues and urine. Anal. Biochem. 1 : 228-239) wurden je 50-100 mg Lebergewebe getrocknet und mit 6N HCl ca. 17 Std gekocht. Nach Verdampfen der Säure im Vakuum-Trockenschrank wurde der Rückstand mit 5 ml Aqua dest. gelöst und filtriert. 200 µl der filtrierten Lösung wurden mit 200 µl Ethanol und 200 µl Oxidationslösung (7 %ige wässrige Chloramin T Hydrat-Lösung 1:4 mit Acetat-CitratPuffer pH 6,0 verdünnt) 25 min bei Zimmertemperatur inkubiert. Danach wurden 400 µl Ehrlich's Reagenz (12g 4-Dimethylaminobenzaldehyd in 20 ml Ethanol + 2,74 ml konzentrierte Schwefelsäure in 20 ml Ethanol) zugegeben. Nach 3 Stunden Inkubation bei 35°C wurde die Absorption bei 573 mn gemessen. Für die Eichreihe wurden wässrige OH-Prolin (Sigma)-Lösungen verwendet. Der OH-Prolin-Gehalt der Leberproben wurde in mg pro g Leber-Trockengewicht berechnet.

### Ergebnisse

Die OH-Prolin-Werte stimmen mit den Ergebnissen der morphometrischen Fibrosemessung sehr gut überein: Die Schweineserum-Behandlung führt ohne gleichzeitige Substanzgabe zu einer ausgeprägten Kollagenakkumulation in der Leber. Die Entstehung dieser Kollagenablagerung wird durch Substanzbehandlung dososabhängig reduziert.

Zur vorliegenden Erfindung gehören pharmazeutische Zubereitungen, die neben nichttoxischen, inerten pharmazeutisch geeigneten Trägerstoffen die erfindungsgemäßen Verbindungen, insbesondere die Verbindungen der allgemeinen Formel (I), enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen, insbesondere die Verbindungen der allgemeinen Formel (I), sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen, insbesondere den Verbindungen der allgemeinen Formel (I), auch weitere pharmazeutische Wirkstoffe enthalten.

Im Allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Die vorliegende Erfindung wird nachstehend anhand von nicht einschränkenden bevorzugten Beispielen näher dargestellt Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- BABA:: n-Butylacetat/n-Butanol/Eisessig/Phosphatpuffer pH 6 (50:9:25.15; org. Phase)

### Laufmittel für die Dünnschichtchromatographie:

- T1 E1:: Toluol - Essigsäureethylester (1:1)
- T1 EtOH1:: Toluol-Methanol (1:1)
- C1 E1:: Cyclohexan - Essigsäureethylester (1: 1)
- C1 E2:: Cyclohexan - Essigsäureethylester (1:2)

### Ausgangsverbindungen

### Bsp. I: 3-(4-Cyclohexylphenoxy)-benzonitril

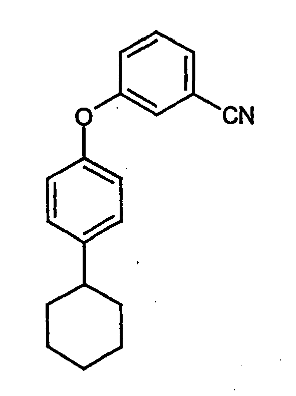

1,2 g (6,81 mmol) 4-Cyclohexylphenol, 7,44 g (40,85 mmol) 3- Brombenzonitril, 1,3 g (6,81 mmol) Kupfer(I)-iodid und 1,88 g (13,62 mmol) Kaliumcarbonat werden unter Argon in 24 ml Pyridin suspendiert und 15 h bei 140°C gerührt. Nach dem Abkühlen wird die Reaktionslösung über Kieselgur filtriert, mit Dichlormethan nachgewaschen und einrotiert. Der erhaltene Rückstand wird in Essigsäureethylester und Wasser aufgenommen und mit 2-*N*-HCl versetzt. Der ausgefallene Niederschlag wird über Kieselgur abfiltriert. Das gewonnene Filtrat wird anschließend zweimal mit 2-*N*-HCl und mit ges. NaCl-Lösung extrahiert. Nach Trocknen über MgSO₄ und Abdampfen des Lösungsmittels wird das Produkt durch Säulenchromatographie (Kieselgel, Cyclohexan/Essigester 25:1) gereinigt. Es werden 876 mg (3,16 mmol, 44 % Ausbeute) eines farblosen Öls erhalten.
R_{f} (Cyclohexan/Essigester, 2:1): 0,71.
¹H-NMR (300 MHz, CDCl₃, δ/ppm): 7,42-7,32 (2H, m), 7,29-7,15 (4H, m), 6,95 (2H, d), 2,53 (1H, m), 1,96-1,60 (5H, m), 1,50-1,25 (5H, m).
MS (DCI, NH₃): 572 (2M+NH₄⁺), 317 (M+N₂H₇⁺), 295 (M+NH₄⁺), 277 (M⁺).

### Bsp. II: 3-(4-Cyclohexylphenoxy)-benzylamin

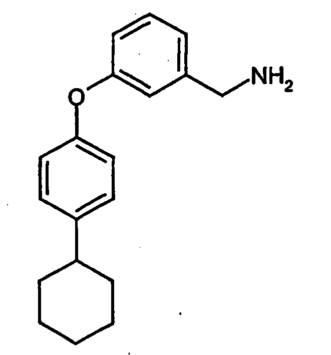

4,32 ml (4,32 mmol) einer 1-molaren Lösung von LiAlH₄ in THF werden bei 0°C tropfenweise mit einer Lösung von 600 mg (2,16 mmol) 3-(4-Cyclohexylphenoxy)-benzonitril aus Bsp. I in 6 ml wasserfreien Diethylether versetzt. Innerhalb von 4 h wird das Reaktionsgemisch auf Raumtemperatur erwärmt und anschließend vorsichtig mit 10 ml ges.-NH₄Cl-Lösung versetzt, mit Ether verdünnt und die organische Phase abgetrennt. Die organische Phase wird nacheinander mit Wasser und ges.-NaCl-Lösung gewaschen, über wasserfreiem MgSO₄ getrocknet und nach Filtration vom Lösemittel befreit. Es werden 573 mg (1,81 mmol, Reinheit 88,82 %, 84 % Ausbeute) 3-(4-Cyclohexylphenoxy)-benzylamin erhalten.
R_{f} (Dichlormethan/Methanol 9/1): 0,13.
¹H-NMR (400 MHz, CDCl₃, δ/ppm): 7,26 (2H, d), 7,16 (2H, d), 7,05-6,85 (4H, m), 3,86 (2H, s), 2,51 (1H, m), 1,93-1,79 (4H, m), 1,70-1,55 (2H, m), 1,48-1,31 (4H, m).
MS (EI): 281 (M⁺).

### Bsp. III: 4-({[3-(4-Cyclohexylphenoxy)-benzyl]-amino}-methyl)-benzoesäuremethylester

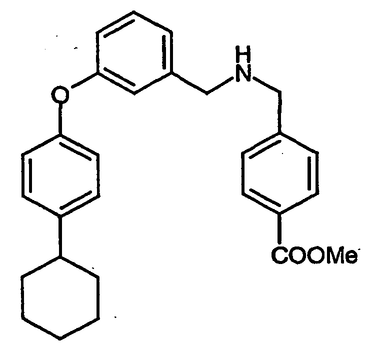

Eine Lösung von 535 mg (1,90 mmol) 3-(4-Cyclohexylphenoxy)-benzylamin aus Bsp. II und 312 mg (1,90 mmol) 4-Formyl-benzoesäuremethylester in 5 ml Dichlormethan werden unter Argon mit 806 mg (3,80 mmol) Natriumtriacetoxyborhydrid versetzt und über Nacht bei Raumtemperatur gerührt. Anschließend wird das Reaktionsgemisch vorsichtig mit 10 ml ges. NaHCO₃-Lösung versetzt, mit Dichlormethan verdünnt und die organische Phase abgetrennt. Die organische Phase wird über MgSO₄ getrocknet und einrotiert. Es werden 429 mg (1 mmol) 4-({[3-(4-Cyclo-hexylphenoxy)-benzyl]-amino}-methyl)-benzoesäure-methylester als farbloses Öl erhalten.
R_{f} (Dichlormethan/Methanol 10:1): 0,56.
¹H-NMR (300 MHz, CDCl₃, δ/ppm): 7,99 (2H, d), 7,40 (2H, d), 7,28 (1H, d), 7,18 (2H, d), 7,08-6,99 (2H, m), 6,97-6,87 (3H, m), 3,91 (3H, s) 3,85 (2H, s), 3,78 (2H, s), 2,51 (1H, m), 1,93-1,70 (5H, m), 1,50-1,32 (5H, m).
MS (ESI): 430 (M+H⁺).

### Bsp. IV: 4-{[[3-(4-Cyclohexylphenoxy)-benzyl]-(5-methoxy-5-oxopentyl)-amino]-methyl}-benzoesäure-methylester

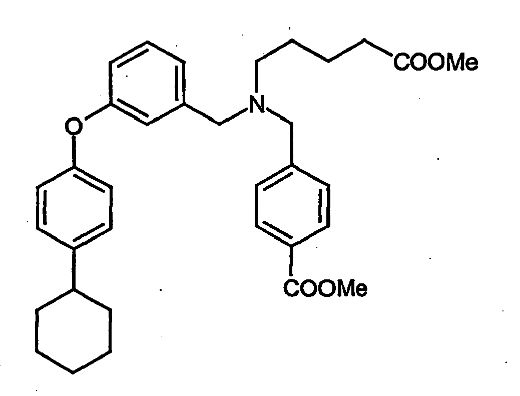

Eine Lösung von 381 mg (0,89 mmol) 4-({[3-(4-Cyclohexylphenoxy)-benzyl]-amino}-methyl)-benzoesäure-methylester aus Bsp. III und 140 µl (0,98 mmol) 5-Bromvaleriansäure-methylester in 3,3 ml Acetonitril wird mit 207 mg (1,95 mmol) wasserfreiem Kaliumcarbonat versetzt und 48 Stunden lang zum Rückfluß erhitzt. Anschließend wird der Ansatz eingedampft, mit Essigester aufgenommen und mit Wasser gewaschen. Nach Trocknen über Na₂SO₄, Filtration und Einengen wird das Produkt durch Säulenchromatographie (Kieselgel, Cyclohexan/Essigester 5:1) gereinigt. Es werden 389 mg (0,71 mmol, 78 % Ausbeute) eines farblosen Öls erhalten.
R_{f} (Dichlormethan): 0,09.
¹H-NMR (300 MHz, DMSO-d₆, δ/ppm): 7,87 (2H, d), 7,39 (2H, d), 7,30 (1H t), 7,23 (2H, d), 7,04 (1H, d), 6,95-6,83 (4H, m), 3,82 (3H, s) 3,60 (5H, s), 3,55 (4H, m), 2,51 (1H, m halb verdeckt durch DMSO), 2,19 (2H, t) 1,90-1,73 (7H, m), 1,70-1,53 (2H, m) 1,45-1,32 (5H, m).
MS (ESI): 544 (M+H⁺).

### Bsp. V: 1-Brom-4-[brom(difluor)methyl]benzol

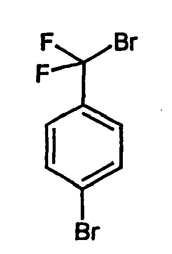

Unter Sauerstoffausschluß wird eine Lösung von 14,0 g (67,63 mmol) 1-Brom-4-(difluormethyl)benzol (CAS 51776-71-7) und 25,3 g (142 mmol) *N*-Bromsuccinimid (NBS) in 190 ml Tetrachlorkohlenstoff mit einer Tageslichtlampe bestrahlt. Dabei erreicht das Lösemittel seine Siedetemperatur. Es wird 24 Stunden am Rückfluss bestrahlt. Anschliessend läßt man auf Raumtemperatur abkühlen und filtriert ausgefallenes Succinimid ab. Das Filtrat wird erneut mit 25 g NBS versetzt und noch einmal für 24 Stunden unter Sauerstoffausschluss am Rückfluss bestrahlt. Nach dem Abkühlen wird wiederum filtriert und das Filtrat zur Trockene eingedampft. Es werden 18 g eines dunkel orange farbenen Öls erhalten, das durch Vakuumdestillation bei 13 Torr gereinigt wird. Es werden 12,7 g (44,4 mmol, 66 % Ausbeute) eines farblosen Öls erhalten.
Siedepunkt (13 Torr): 90-92°C.
¹H-NMR (300 MHz, DMSO-d₆, δ/ppm): 7,79 (2H, d), 7,63 (2H, d).
MS (ESI): 205/207 (M-Br⁻).

### Bsp. VI: 4-({[2-(2-Methoxyphenyl)ethyl]amino}methyl)benzoesäure-methylester

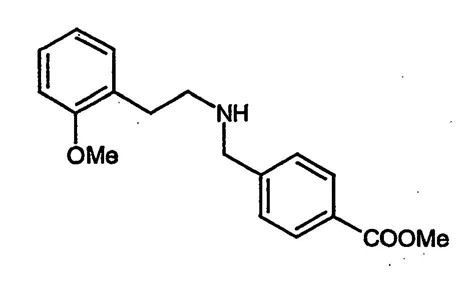

Eine Lösung von 92,08 g (0,597 mol) 2-Methoxyphenethylamin und 98,0 g (0,597 mol) 4-Formylbenzoesäure-methylester in 2 1 Ethanol wird 2 Stunden zum Rückfluss erhitzt. Anschliessend wird das Lösemittel im Vakuum abgezogen und der erhaltene Rückstand in 11 Methanol gelöst. Portionsweise werden insgesamt 46,14 g fester NaBH₄ zugesetzt. Nach zwei Stunden Rühren bei Raumtemperatur wird der Ansatz in Wasser gegossen und mit Essigester extrahiert. Der organische Extrakt wird mit gesättigter Kochsalz-Lösung gewaschen und über Na₂SO₄ getrocknet. Nach Filtration wird das Lösemittel im Vakuum entfernt. Es werden 167,7 g (0,559 mol, 77 % Ausbeute) eines farblosen Öls erhalten, das ohne weitere Reinigung in der nächsten Stufe eingesetzt wird.
¹H-NMR (200 MHz, DMSO-d₆, δ/ppm): 7,90 (2H, d), 7,45 (2H, d), 7,17 (1H, t), 7,12 (1H, d), 6,92 (1H, d), 6,83 (1H, t), 3,83 (3H, s), 3,78 (2H, s), 3,73 (3H, s), 2,75-2,63 (4H, m).
MS (DCI, NH₃): 300 (M+H⁺).

### Bsp. VII: 4-({[2-(2-Hydroxyphenyl)ethyl]amino}methyl)benzoesäure-methylester-Hydrobromid

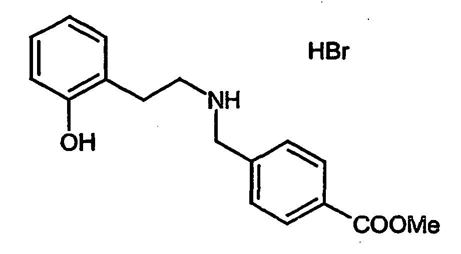

Eine Lösung von 60,0 g (0,2 mol) 4-({[2-(2-Methoxyphenyl)ethyl]amino}methyl)-benzoesäure-methylester aus Bsp. VI in 200 ml Dichlormethan wird bei 0°C mit 661,4 ml (0,66 mol) einer 1-molaren Lösung von Bortribromid in Dichlormethan versetzt. Man läßt eine Stunde bei 0°C weiterrühren. Dann werden 300 ml Methanol zugefügt und der Ansatz 18 Stunden zum Rückfluss erhitzt. Beim Abkühlen fällt das Produkt aus und wird abfiltriert. Weiteres Produkt wird nach Aufkonzentrieren der Mutterlauge erhalten. Die gesammelten Produktfraktionen werden mit Ether gewaschen. Es werden 45,04 g (0,16 mol, 56 % Ausbeute) eines weissen kristallinen Feststoffs erhalten.
R_{f} (Dichlormethan/Methanol 10:1): 0,54.
¹H-NMR (300 MHz, DMSO-d₆, δ/ppm): 9,58 (1H, breit), 9,02 (2H, breit), 8,03 (2H, d), 7,68 (2H, d), 7,09 (1H, d), 7,07 (1H, t), 6,82 (1H, d), 6,77 (1H, t), 4,29 (2H, s), 3,89 (3H, s), 3,18-3,10 (2H, m), 2,94-2,88 (2H, m).
MS (ESI): 286 (M+H⁺).

### Bsp. VIII: 4-{[[2-(2-Hydroxyphenyl)ethyl](5-methoxy-5-oxopentyl)amino]-methyl}benzoesäure-methylester

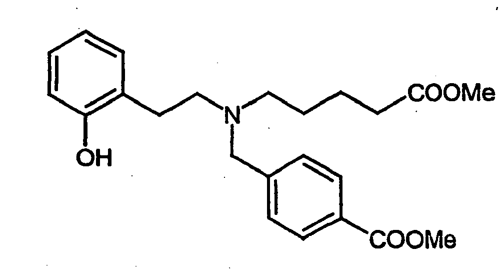

3,0 g (8,19 mmol) 4-({[2-(2-Hydroxyphenyl)ethyl]amino}methyl)benzoesäuremethylester-Hydrobromid aus Bsp. VII, 1,3 ml (9,83 mmol) 5-Bromvaleriansäuremethylester und 1,74 g (16,38 mmol) wasserfreies Natriumcarbonat werden in 20 ml Acetonitril drei Tage zum Rückfluss erhitzt. Anschliessend wird der Ansatz zur Trockene eingedampft, der Rückstand mit Essigester aufgenommen und mit Wasser und gesättigter Kochsalz-Lösung gewaschen. Nach Trocknen über Na₂SO₄ wird filtriert und eingedampft. Das Produkt wird durch Flash-Chromatographie (Kieselgel, Cyclohexan/Essigester 7:3) gereinigt. Es werden 2,2 g (5,51 mmol, 67 % Ausbeute) eines blass gelben Öls erhalten.
R_{f} (Cyclohexan/Essigester 2:1): 0,28.
¹H-NMR (300 MHz, DMSO-d₆, δ/ppm): 9,57 (1H, s breit), 7,89 (2H, d), 7,43 (2H, d), 6,99 (1H, d), 6,98 (1H, t), 6,72 (1H, d), 6,67 (1H, t), 3,83 (3H, s), 3,69 (2H, s), 3,57 (3H, s), 2,71-2,66 (2H, m), 2,62-2,55 (2H, m), 2,45 (2H, t), 2,23 (2H, t), 1,51-1,40 (4H, m).
MS (DCI, NH₃): 400 (M+H⁺), 252.

### Bsp. IX: 4-{[(2-{2-[(4-Bromphenyl)(aifluor)methoxy]phenyl}ethyl)(5-methoxy-5oxopentyl)amino]methyl}benzoesäure-methylester

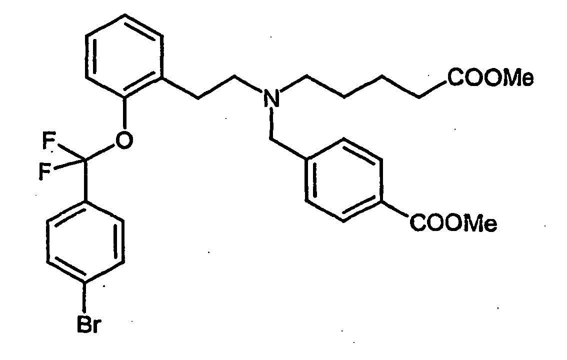

240 mg (6,0 mmol) einer 60%igen Suspension von NaH in Mineralöl werden in 60 ml wasserfreiem DMF vorgelegt und bei 0°C mit einer Lösung von 2,0 g (5,0 mmol) 4-{[[2-(2-Hydroxyphenyl)ethyl](5-methoxy-5-oxopentyl)amino]methyl}-benzoesäuremethylester aus Bsp. VIII in 1 ml DMF versetzt. Man läßt das Gemisch auf Raumtemperatur kommen. Nach 30 Minuten werden 1,59 g (5,0 mmol) 1-Brom-4-[brom(difluor)methyl]benzol aus Bsp. V, gelöst in 1 ml DMF, zugetropft und das Gemisch auf 70°C erwärmt. Nach 15 Stunden läßt man wieder auf Raumtemperatur abkühlen, verdünnt mit Dichlormathan und wäscht nacheinander mit wässriger 5 %iger NaH₂PO₄-Lösung und gesättigter Kochsalzlösung. Trocknen über Na₂SO₄. Das Produkt wird durch Flash-Chromatographie (Kieselgel, Cyclohexan/Essigester-Gradient 20;1 → 5:1) gereinigt. Es werden 1,42 g (2,35 mmol, 47 % Ausbeute) eines zähen, gelben Öls erhalten.
R_{f} (Cyclohexan/Essigester 2:1): 0,39.
¹H-NMR (300 MHz, DMSO-d₆, δ/ppm): 7,82 (2H, d), 7,76 (2H, d), 7,65 (2H, d), 7,33 (2H, d), 7,30-7,18 (4H, m), 3,86 (3H, s), 3,59 (2H, s), 3,57 (3H, s), 2,77 (2H, dd), 2,58 (2H, dd), 2,48 (2H, t), 2,14 (2H, t), 1,43-1,28 (4H, m).
MS (DCI, NH₃): 604/606 (M+H⁺).

### Bsp. X: 4-{[[2-(2-{Difluoro[4'-(trifluormethyl)-1,1'-biphenyl-4-yl]methoxy}-phenyl)ethyl](5-methozy-5-oxopentyl)amino]methyl}benzoesäure-methylester

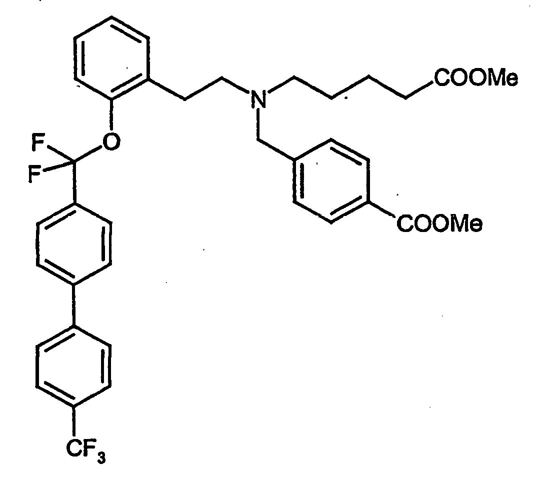

Eine Lösung von 300 mg (0,50 mmol) 4-{[(2-{2-[(4-Bromphenyl)(difluor)methoxy]-phenyl}ethyl)(5-methoxy-5-oxopentyl)amino]methyl}benzoesäure-methylester aus Bsp. IX, 103,4 mg (0,55 mmol) 4-Trifluormethylbenzolboronsäure und 17,2 mg (0,01 mmol) Tetrakis(triphenylphosphino)palladium-(0) in 5 ml 1,2-Dimethoxyethan wird mit 0,75 ml einer 2-molaren, wässrigen Natriumcarbonat-Lösung versetzt und 18 Stunden unter Argon zum Rückfluss erhitzt. Anschliessend wird mit Essigester verdünnt und nacheinander mit 5 %iger NaH₂PO₄-Lösung, Wasser und gesättigter Kochsalz-Lösung gewaschen. Trocknen über Na₂SO₄. Das Produkt wird durch Flash-Chromatographie (Kieselgel, Cyclohexan/Essigester-Gradient 30:1 → 1:1) gereinigt. Es werden 260 mg (0,39 mmol, 78 % Ausbeute) eines gelben Öls erhalten.
R_{f} (Cyclohexan/Essigester 4:1): 0,28.
¹H-NMR (300 MHz, DMSO-d₆, δ/ppm): 7,95-7,79 (10H, m), 7,37-7,19 (6H, m), 3,79 (3H, s), 3,60 (2H, s), 3,49 (3H, s), 2,81 (2H, dd), 2,61 (2H, dd), 2,40 (2H, t), 2,12 (2H, t), 1,41-1,30 (4H, m).
MS (ESI): 670 (M+H⁺).

### Bsp. XI: 2-[(4-Cyclohexylphenoxy)methyl]-5-fluorbenzoesäure-methylester

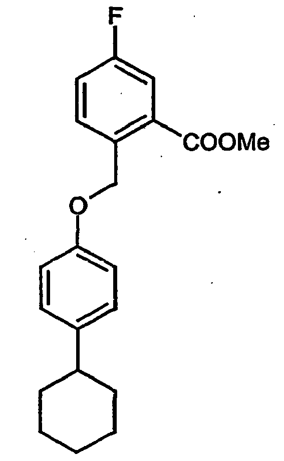

2,14 g (12,14 mmol) 4-Cyclohexylphenol und 3,0 g (12,14 mmol) 2-(Brommethyl)-5-fluorbenzoesäure-methylester (CAS 138786-65-9) werden zusammen mit 2,5 g (18,21 mmol) wasserfreiem Kaliumcarbonat in 20 ml Acetonitril zum Rückfluss erhitzt. Nach drei Stunden wird der Ansatz zur Trockene einrotiert. Der Rückstand wird in Ether aufgenommen und nacheinander mit Wasser und gesättigter Kochsalz-Lösung gewaschen. Das Trocknen wirde über Na₂SO₄ durchgeführt. Das Rohprodukt wird durch Flash-Chromatographie (Kieselgel, Cyclohexan/Essigester 80:1) gereinigt. Es werden 3,37 g (9,84 mmol, 81 % Ausbeute) eines farblosen Öls erhalten.
R_{f} (Cyclohexan/Essigester 9:1): 0,56.
¹H-NMR (200 MHz, DMSO-d₆, δ/ppm): 7,72-7,43 (3H, m), 7,13 (2H, d), 6,88 (2H, d), 5,32 (2H, s), 3,81 (2H, s), 2,51-2,36 (1H, m), 1,80-1,62 (5H, m), 1,46-1,20 (5H, m).
MS (ESI): 707 (2M+Na⁺), 365 (M+Na⁺).

### Bsp. XII: {2-[(4-Cyclohexylphenoxy)methyl]-5-fluorphenyl}methanol

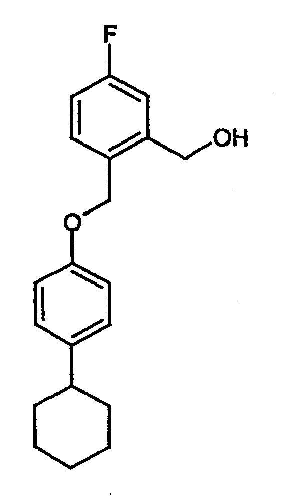

6,6 ml (6,6 mmol) einer 1-molaren Lösung von LiAlH₄ in Ether werden vorgelegt und mit weiteren 20 ml Ether verdünnt. Ohne Kühlung des Reaktionskolbens läßt man eine Lösung von 3,2 g (9,35 mmol) 2-[(4-Cyclohexylphenoxy)methyl]-5-fluorbenzoesäure-methylester aus Bsp. XI in 20 ml Ether so zutropfen, dass das Reaktionsgemisch gerade zum Sieden kommt. Nach 30 Minuten wird das Reaktionsgemisch mit Ether verdünnt und vorsichtig mit 20 %iger, wässriger Kalium-natriumtartrat-Lösung versetzt. Die organische Phase wird abgetrennt und nacheinander mit Wasser und gesättigter Kochsalz-Lösung gewaschen. Trocknen über Na₂SO₄. Nach Filtration und Einrotieren werden 2,57 g (8,17 mmol, 87 % Ausbeute) Produkt als weisser Feststoff erhalten.
R_{f} (Cyclohexan/Essigester 9:1): 0,13.
¹H-NMR (300 MHz, DMSO-d₆, δ/ppm): 7,45 (1H, dd), 7,27 (1H, dd), 7,12 (2H, d), 7,06 (1H, dt), 6,91 (2H, d), 5,31 (1H, t), 5,03 (2H, s), 4,60 (2H, d), 2,48-2,38 (1H, m), 1,80-1,66 (5H, m), 1,42-1,18 (5H, m).
MS (DCI, NH₃): 646 (2M+NH₄⁺), 332 (M+NH₄⁺).

### Bsp. XIII: 2-(Brommethyl)-1-[(4-cyclohexylphenoxy)methyl]-4-fluorbenzol

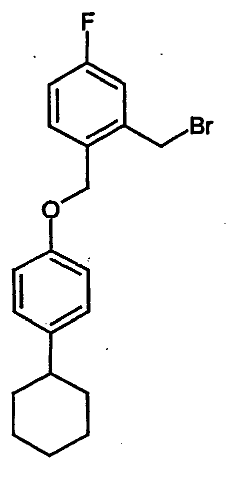

Eine Lösung von 2,5 g (9,54 mmol) Triphenylphosphin und 3,2 g (9,54 mmol) Tetrabrommethan in 30 ml Ether wird mit einer Lösung von 2,5 g (7,95 mmol) {2-[(4-Cyclohexylphenoxy)methyl]-5-fluorphenyl}methanol aus Bsp. XII in 30 ml Ether versetzt. Nach 20 Stunden rühren bei Raumtemperatur werden weitere 0,83 g Triphenylphosphin und 1,05 g Tetrabrommethan fest hinzugegeben. Nach sechs Stunden wird der Ansatz zur Trockene eingedampft und das Produkt durch Flash-Chromatographie (Kieselgel, Cyclohexan/Essigester 50:1) isoliert. Es werden 2,3 g (6,1 mmol, 77 % Ausbeute) eines farblosen, tief schmelzenden Feststoffs erhalten.
R_{f} (Cyclohexan/Essigester 9:1): 0,50.
¹H-NMR (300 MHz, DMSO-d₆, δ/ppm): 7,52 (1H, dd), 7,39 (1H, dd), 7,20 (1H, dt), 7,15 (2H, d), 6,95 (2H, d), 5,17 (2H, s), 4,79 (2H, s), 2,50-2,39 (1H, m), 1,80-1,65 (5H, m), 1,42-1,18 (5H, m).
MS (EI+): 376/378 (M⁺).

### Bsp. XIV: {2-[(4-Cyclohexylphenoxy)methyl]-5-fluorphenyl}acetonitril

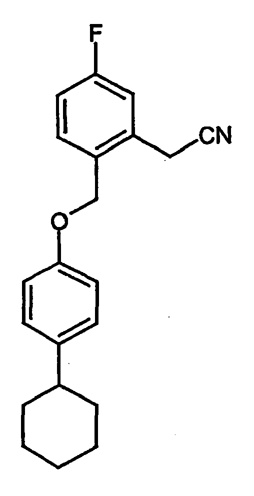

Eine Lösung von 0,8 ml (5,96 mmol) Trimethylsilylcyanid in 5 ml Acetonitril wird mit 6 ml (5,96 mmol) einer 1-molaren Lösung von Tetra-*n*-butylammoniumfluorid in THF versetzt. Nach fünf Minuten wird eine Lösung von 1,5 g (3,98 mmol) 2-(Brommethyl)-1-[(4-cyclohexylphenoxy)methyl]-4-fluorbenzol aus Bsp. XIII in 5 ml Acetonitril zugefügt. Man läßt das Reaktionsgemisch 30 Minuten bei Raumtemperatur rühren. Dann wird der Ansatz vollständig einrotiert und das Produkt durch Flash-Chromatographie (Kieselgel, Cyclohexan/Essigester 20:1) isoliert. Es werden 1,15 g (3,56 mmol, 89 %Ausbeute) eines weissen Feststoffs erhalten.
R_{f} (Cyclohexan/Essigester 4:1): 0,53.
¹H-NMR (300 MHz, DMSO-d₆, δ/ppm): 7,57 (1H, dd), 7,31 (1H, dd), 7,22 (1H, dt), 7,14 (2H, d), 6,95 (2H, d), 5,10 (2H, s), 4,11 (2H, s), 2,48-2,39 (1H, m), 1,80-1,66 (5H, m), 1,42-1,18 (5H, m).
MS (DCI, NH₃): 664 (2M+NH₄⁺), 358 (M+NH3+NH₄⁺), 341 (M+NH₄⁺).

### Bsp. XV: 2-{2-[(4-Cyclohexylphenoxy)methyl]-5-fluorphenyl}ethylamin

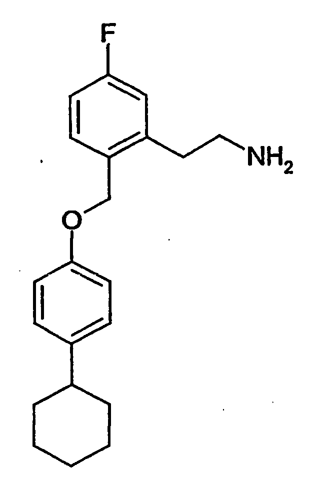

810 mg (2,50 mmol) {2-[(4-Cyclohexylphenoxy)methyl]-5-fluorphenyl}acetonitril aus Bsp. XIV werden in 20 ml wasserfreiem THF vorgelegt und mit 2,53 ml (5,01 mmol) einer 2-molaren Lösung von Boran-Dimethylsulfid-Komplex in THF versetzt. Das Gemisch wird zwei Stunden zum Rückfluss erhitzt. Dann wird der Ansatz abgekühlt, mit verdünnter Salzsäure sauer gestellt und erneut kurz zum Rückfluss erhitzt. Anschliessend läßt man wieder abkühlen und es wird mit verdünnter Natronlauge alkalisch gestellt. Es wird mit Ether extrahiert. Die organische Phase wird nacheinander mit Wasser und gesättigter Kochsalz-Lösung gewaschen. Trocknen über Na₂SO₄. Nach Filtration und Einengen werden 820 mg (2,5 mmol, 100 % Ausbeute) eines blass gelben Öls erhalten, das ohne weitere Reinigung in der nächsten Stufe eingesetzt wird.
R_{f} (Essigester/Methanol 7:3): 0,12.
¹H-NMR (300 MHz, DMSO-d₆, δ/ppm): 7,45 (1H, dt), 7,15-7,00 (4H, m), 6,92 (2H, d), 5,02 (2H, s), 2,78-2,70 (4H, m), 2,50-2,40 (1H, m), 1,92 (2H, breit), 1,80-1,65 (5H, m), 1,42-1,20 (5H, m).
MS (DCI, NH₃): 328 (M+H⁺).

### Bsp. XVI: 4-{[(2-{2-[(4-Cyclohexylphenoxy)methyl]-5-fluorphenyl}ethyl)amino]-methyl}benzoesäure-methylester

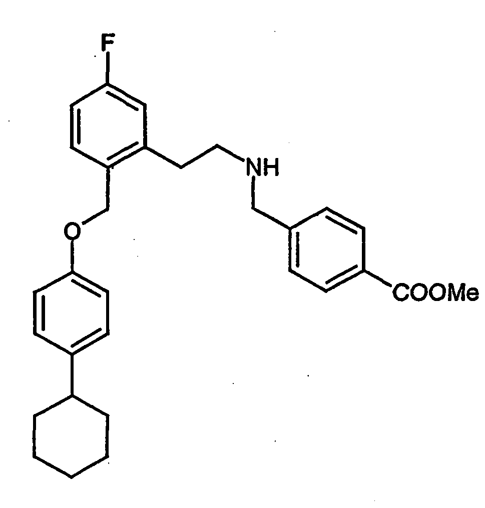

700 mg (2,14 mmol) 2-{2-[(4-Cyclohexylphenoxy)methyl]-5-fluorphenyl}ethylamin aus Bsp. XV und 316 mg (1,92 mmol) 4-Formylbenzoesäure-methylester werden in 50 ml Toluol 30 Minuten am Wasserabscheider gekocht. Anschliessend wird der Ansatz eingedampft und der Rückstand in 20 ml Methanol aufgenommen. Unter EisKühlung werden portionsweise 81 mg (2,14 mmol) festes NaBH₄ zugefügt. Man läßt 30 Minuten bei Raumtemperatur rühren. Dann wird mit 5%iger wässriger NaH₂PO₄-Lösung neutralisiert, mit Wasser verdünnt und mit Ether extrahiert. Die organische Phase wird nacheinander mit Wasser und gesättigter Kochsalz-Lösung gewaschen. Trocknen über Na₂SO₄. Das Produkt wird durch Flash-Chromatographie (Kieselgel, Cyclohexan/Essigester 3:1) isoliert. Es werden 730 mg (1,53 mmol, 80% Ausbeute) eines farblosen Öls erhalten.
R_{f} (Cyclohexan/Essigester 1:2): 0,40.
¹H-NMR (300 MHz, DMSO-d₆, δ/ppm): 7,87 (2H, d), 7,45-7,39 (3H, m), 7,12-7,08 (3H, m), 7,02 (1H, dt), 6,85 (2H, d), 5,00 (2H, s), 3,83 (3H, s), 3,76 (2H, s), 2,83-2,70 (4H, m), 2,47-2,39 (1H, m), 1,74 (1H, s breit), 1,80-1,64 (5H, m), 1,39-1,18 (5H, m).
MS (ESI): 476 (M+H⁺).

### Bsp. XVII: 4-{[(2-{2-[(4-Cyclohexylphenoxy)methyl]-5-fluorphenyl}ethyl)(5-methozy-5-oxopentyl)amino]methyl}benzoesäure-methylester

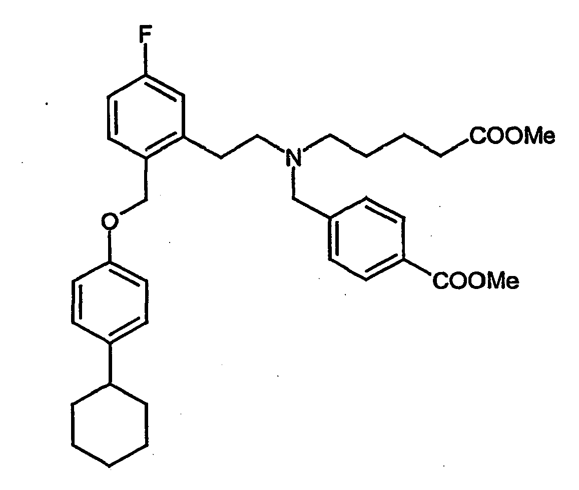

720 mg (1,51 mmol) 4-{[(2-{2-[(4-Cyclohexylphenoxy)methyl]-5-fluorphenyl}-ethyl)amino]methyl}benzoesäure-methylester aus Bsp. XVI, 242 µl (1,82 mmol) 5-Bromvaleriansäure-methylester und 193 mg (1,82 mmol) wasserfreies Natriumcarbonat werden in 20 ml Butyronitril zum Rückfluss erhitzt. Nach 48 Stunden wird der Ansatz eingedampft, mit Essigester aufgenommen und nacheinander mit Wasser und gesättigter Kochsalzlösung gewaschen. Trocknen über Na₂SO₄. Das Produkt wird durch Flash-Chomatographie (Kieselgel, Cyclohexan/Essigester 9:1) gereinigt. Es werden 570 mg (0,97 mmol, 64 % Ausbeute) eines farblosen Öls erhalten.
R_{f} (Cyclohexan/Essigester 2:1): 0,56.
¹H-NMR (300 MHz, DMSO-d₆, δ/ppm): 7,83 (2H, d), 7,42 (1H, dd), 7,37 (2H, d), 7,10 (2H, d), 7,08-6,99 (2H, m), 6,83 (2H, d), 4,93 (2H, s), 3,83 (3H, s), 3,62 (2H, s), 3,55 (3H, s), 2,80 (2H, dd), 2,63 (2H, dd), 2,48-2,37 (3H, m), 2,15 (2H, t), 1,80-1,65 (5H, m), 1,42-1,14 (9H, m).
MS (ESI): 590 (M+H⁺).

### Synthesebeispiele

### Bsp. 1: 4-({(4-Carboxybutyl)[3-(4-cyclohexylphenoxy)-benzyl]-amino}-methyl)-benzoesäure

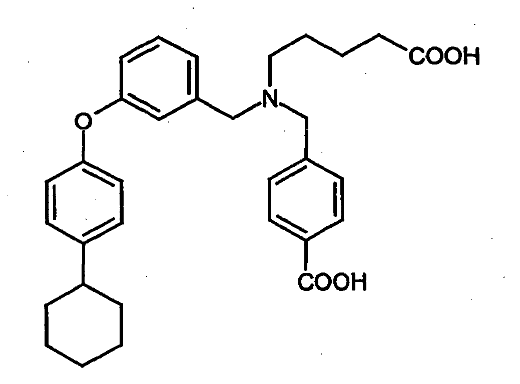

Eine Lösung von 352 mg (0,65 mmol) 4-{[[3-(4-Cyclohexylphenoxy)-benzyl]-(5-methoxy-5-oxopentyl)-amino]-methyl}-benzoesäure-methylester aus Bsp. IV in 3,5 ml Dioxan und 1,8 ml Wasser wird mit 195 µl einer 45 %igen Lösung von NaOH in Wasser versetzt und 2 Stunden lang bei 90°C gerührt. Nach dem Abkühlen wird das Dioxan abgezogen und die wässrige Phase wird mit 1-molarer Salzsäure auf pH 4 bis 5 eingestellt. Dabei fällt das Produkt aus, das abfiltriert, mit Wasser gewaschen und getrocknet wird. Es werden 280 mg (0,54 mmol, 83 % Ausbeute) eines weißen Feststoffs erhalten.
R_{f} (Essigester/Methanol 7:3): 0,38.
¹H-NMR (300 MHz, DMSO-d₆, δ/ppm): 12,49 (2H, breites s), 7,89 (2H, d), 7,47 (2H, d), 7,29 (1H t), 7,20 (2H, d), 7,05 (1H, d), 6,98-6,80 (4H, m), 3,55 (2H, s) 3,50 (2H, s), 2,51 (1H, m halb verdeckt durch DMSO), 2,41 (2H, m), 2,08 (2H, m) 1,89-1,62 (6H, m), 1,49-1,13 (8H, m).
MS (ESI): 1030 (2M+H+), 516 (M+H⁺).

Auf analoge Weise wurde erhalten:

| Bsp. | Formel | ¹H-NMR δ [ppm] (DMSO-d₆) |
|---|---|---|
| 2 (ausgehend von 4-(4- Trifluormethylphenyl)- phenol) | 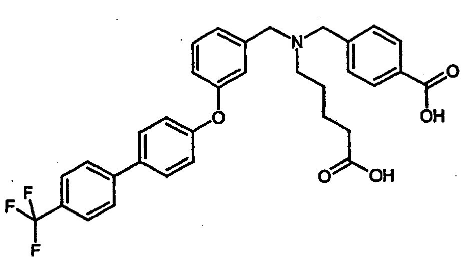 | 12,36 (2H, breit), 7,79- 7,69 (8H, m), 7,45-7,40 (3H, m), 7,21-7,01 (4H, m), 6,95 (1H, d), 3,59 (2H, s), 3,51 (2H, s), 2,41-2,29 (2H,m), 2,17- 2,02 (2H, m), 1,55-1,34 (4H, m). (300 MHz) |

### Bsp. 3: 4-({(4-Carbozybutyl)[3-(4-cyclohexylphenoxy)benzyl]amino}methyl)-benzoesäure-Hydrochlorid

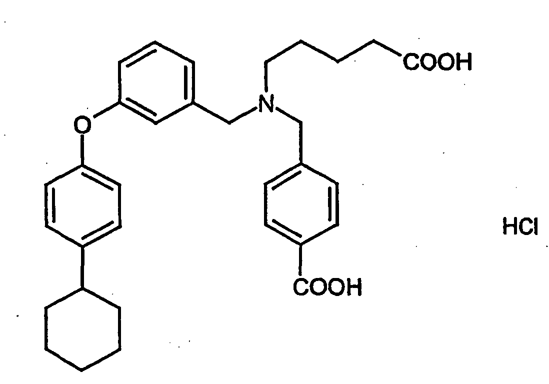

Eine Lösung von 220 mg (0,43 mmol) 4-({(4-Carboxybutyl)[3-(4-cyclohexyl-phenoxy)-benzyl]-amino}-methyl)-benzoesäure aus Bsp. 1 in 0,2 ml Dioxan werden mit 0,5 ml (2 mmol) einer 4-molaren Lösung von HCl in Dioxan versetzt und 1 h bei 60°C gerührt. Anschließend wird der Ansatz eingedampft und das erhaltene farblose Öl mehrmals mit Diethylether verrührt. Die entstandenen Kristalle werden filtriert und getrocknet. Es werden 171 mg (0,31 mmol, 72% Ausbeute) eines weißen Feststoffs erhalten.

R_{f} (Essigester/Methanol 7:3): 0,42.
¹H-NMR (300 MHz, DMSO-d₆, δ/ppm): 12,50 (2H, breites s), 10,47 (1H, breites s), 7,99 (2H, d), 7,66 (2H, d), 7,49 (1H t), 7,35 (1H, d), 7,25 (3H, d), 7,06 (1H, d) 6,95 (2H, m), 3,40 (2H, d) 3,45 (2H, d), 2,92 (2H, m) 2,51 (1H, m halb verdeckt durch DMSO), 2,20 (2H, t), 1,89-1,62 (7H, m), 1,49-1,15 (7H, m). MS (ESI): 516 (M+H⁺-HCl).

Auf analoge Weise wurden erhalten:

| Bsp. | Formel | ¹H-NMR δ [ppm] (DMSO-d₆) |
|---|---|---|
| 4 (aus 2) | 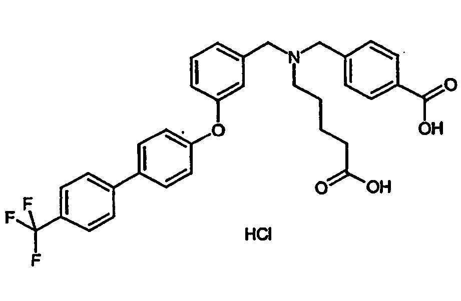 | 12,5 (2H, breit), 10,45 (1H, breit), 7,96 (2H,d), 7,88(2H, d), 7,84-7,72 (3H, m), 7,65 (2H, d), 7,55-7,18 (4H, m), 7,15 (2H,d), 6,93 (1H, d), 4,43-4,28 (4H, m), 3,02- 2,88(2H,m), 2,19 (2H,t), 1,88-1,66 (2H, m), 1,50- 1,32 (2H,m). (300 MHz) |
| 5 (ausgehend von 4-(4- Fluorphenyl)- phenol) | 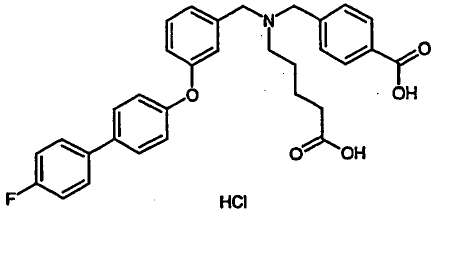 | 12,74 (2H, breit), 10,79 (1H, breit), 7,97 (2H, d), 7,78-7,57 (6H, m), 7,53- 7,02 (8H, m), 4,49-4,21 (4H, m), 3,02-2,80 (2H, m), 2,20 (2H, t), 1,88 (2H, m), 1,51-1,28 (2H, m). (200 MHz) |

### Bsp. 6: 4-({(4-Carboxybutyl)[2-(2-{difluor[4'-(trifluormethyl)-1,1'-biphenyl-4-yl]methoxy}phenyl)ethyl]amino}methyl)benzoesäure

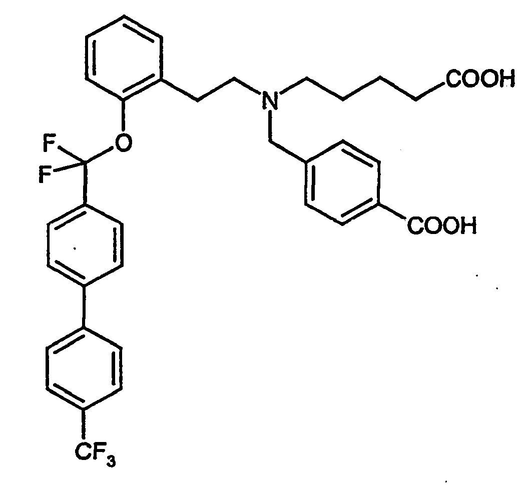

125 mg (0,19 mmol) 4-{[[2-(2-{Dilluoro[4'-(trifluormethyl)-1,1'-biphenyl-4-yl]-methoxy}phenyl)ethyl](5-methoxy-5-oxopentyl)amino]methyl}benzoesäure-methylester aus Bsp. X werden in einer Mischung aus 2 ml THF, 1 ml Methanol und 3 ml 2-molarer wässriger LiOH-Lösung 1,5 Stunden auf 60°C erwärmt. Anschliessend werden die organischen Lösemittel weitgehend am Rotationsverdampfer entfernt. Die erhaltene wässrige Lösung wird zunächst mit Ether gewaschen und dann mit 1-molarer Salzsäure auf pH 4 bis 5 eingestellt. Es wird mit Essigester extrahiert. Die organische Phase wird über Na₂SO₄ getrocknet. Nach Filtration und Eindampfen werden 107 mg Rohprodukt erhalten, die per HPLC gereinigt werden. Die Produktfraktionen werden vereinigt, und es wird aus Methanol umkristallisiert. Es werden 83 mg (0,13 mmol) eines weissen Feststoffs erhalten.
R_{f} (Essigester/Methanol 7:3): 0,48.
¹H-NMR (200 MHz, DMSO-d₆, δ/ppm): 12,38 (2H, breit), 7,98-7,77 (10H, m), 7,37-7,18 (6H, m), 3,59 (2H, s), 2,81 (2H, dd), 2,61 (2H, dd), 2,41 (2H, t), 2,08 (2H, t), 1,44-1,32 (4H, m).
MS (ESI): 642 (M+H⁺).

Auf analoge Weise wurden hergestellt:

| Bsp. | Formel | ¹H-NMR δ [ppm] (DMSO-d₆) |
|---|---|---|
| 7 (analog 6, doch unter Verwendung von Benzolboronsäure) | 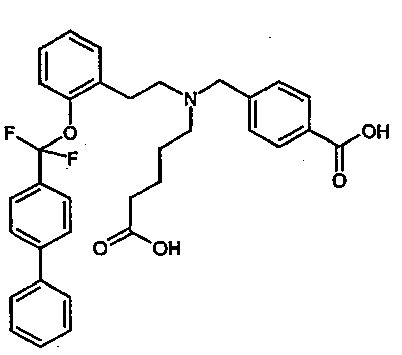 | 12,08 (2H), 7,83-7,77 (6H, m), 7,71 (2H, d), 7,52-7,41 (3H, m), 7,37-7,18 (6H, m), 3,61 (2H, s), 2,82 (2H, m), 2,61 (2H, m), 2,42 (2H, m), 2,08 (2H, m), 1,40 (4H, m). (300 MHz) |
| 8 (analog 6, doch unter Verwendung von 4- t-Butyl benzolboronsäure) | 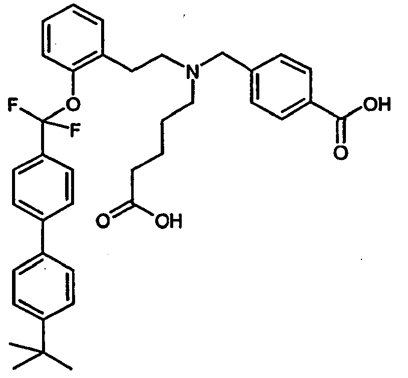 | 12,22 (2H, breit), 7,82-7,74 (6H, m), 7,63 (2H, d), 7,51 (2H, d), 7,33-7,28 (5H, m), 7,26-7,18 (1H, m), 3,60 (2H, s), 2,81 (2H, dd), 2,61 (2H, dd), 2,41 (2H, t), 2,09 (2H, t), 1,42-1,37 (4H, m), 1,33 (9H, s). (300 MHz) |
| 9 (analog 6, doch unter Verwen dung von 4- Chlorbenzolboronsäure) | 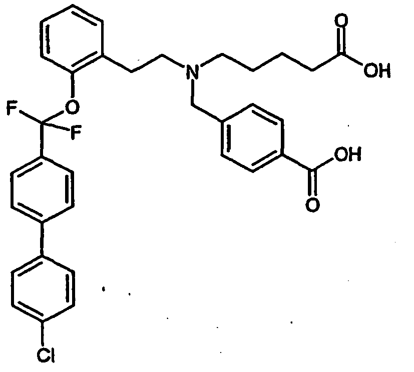 | 12,42 (2H, breit), 7,87-7,72 (8H, m), 7,56 (2H, d), 7,33- 7,18 (6H, m), 3,60 (2H, s), 2,81 (2H, dd), 2,60 (2H, dd), 2,43-2,37 (2H, m), 2,12-2,04 (2H, m), 1,42- 1,34 (4H, m). (200 MHz) |
| 10 (analog 6, doch unter Verwendung von 4- Methoxybenzolboronsäure) | 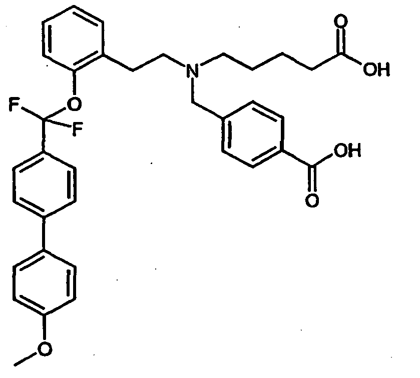 | 12,40 (2H, breit), 7,87-7,63 (8H, m), 7,39-7,20 (6H, m), 7,07 (2H, d), 3,82 (2H, s), 3,61 (2H, breit), 2,82 (2H, breit), 2,62 (2H, breit), 2,41 (2H, breit), 2,10 (2H, breit), 1,39 (4H, breit). (200 MHz) |

### Bsp. 11: 4-({(4-Carboxybutyl)[2-(2-{difluor[-1,1'-biphenyl-4-yl]methoxy}-phenyl)ethyl]amino}methyl)benzoesäure Hydrochlorid

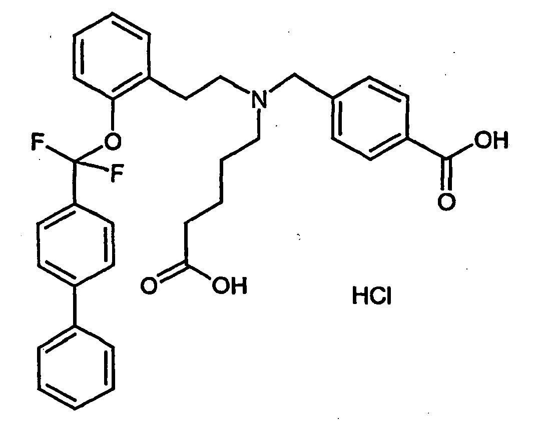

Diese Verbindung wurde ausgehend von Bsp. 7 analog zu Bsp. 3 hergestellt.
¹H-NMR: δ [ppm] (DMSO-d₆):12,70 (2H, breit), 10,49 (1H, breit), 7,97 (2H, d), 7,83 (4H, s), 7,73-7,68 (4H, m), 7,57-7,29 (7H, m), 4,47 (2H, s breit), 3,20-3,15 (6H, m), 2,18 (2H, t), 1,79-1,63 (2H, m), 1,52-1,49 (2H, m). (200 MHz)

### Bsp. 12: 4-{[(4-Carboxybutyl)(2-{2-[(4-cyclohexylphenoxy)methyl]-5-fluorphenyl}ethyl)amino]methyl}benzoesäure

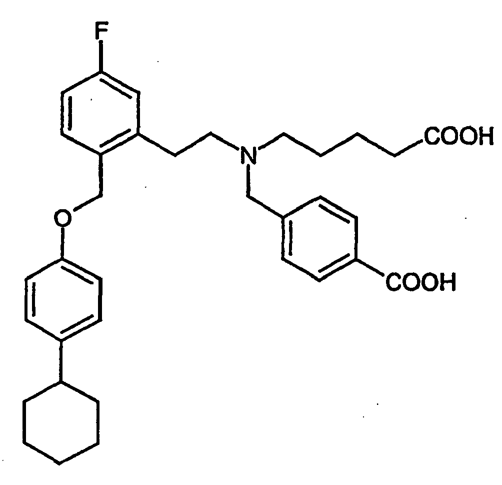

500 mg (0,85 mmol) 4-{[(2-{2-[(4-Cyclohexylphenoxy)methyl]-5-fluorphenyl}-ethyl)(5-methoxy-5-oxopentyl)amino]methyl}benzoesäure-methylester aus Bsp. XVII werden in 5 ml THF gelöst und mit 20 ml 2-molarer Natronlauge versetzt. Es wird 15 Stunden auf 50 bis 60°C erwärmt. Nach dem Abkühlen wird mit Ether extrahiert und anschliessend die wässrige Phase mit 2-molarer Salzsäure auf pH 4 bis 5 gestellt. Das Produkt fällt dabei in Form eines weissen Feststoffs aus, der abgesaugt und mit Wasser gewaschen wird. Es werden 420 mg (0,75 mmol, 88 % Ausbeute) erhalten. Schmelzpunkt: >250°C.
R_{f} (Essigester/Methanol 7:3): 0,43.
¹H-NMR (300 MHz, DMSO-d₆, δ/ppm): 12,52 (2H, breit), 7,81 (2H, d), 7,42 (1H, dd), 7,27 (2H, d), 7,11 (2H, d), 7,08-6,98 (2H, m), 6,84 (2H, d), 4,93 (2H, s), 3,59 (2H, s), 2,79 (2H, dd), 2,63 (2H, dd), 2,48-2,37 (3H, m), 2,11-2,04 (2H, m), 1,79-1,63 (5H, m), 1,41-1,16 (9H, m).
MS (ESI): 562 (M+H⁺).

Auf analoge Weise wurden erhalten:

| Bsp. | Formel | Analytische Daten |
|---|---|---|
| 13 (analog 12, doch unter Verwendung von 4-t-Butylphenol) | 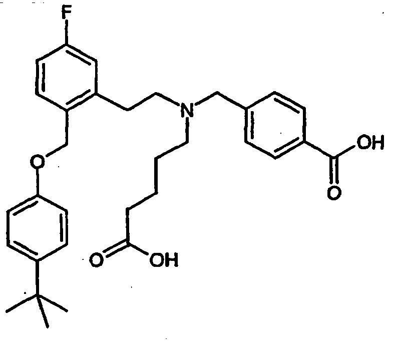 | Schmelzpunkt (°C): >250 MS (ESI): 536 (M+H⁺) |
| 14 (analog 12, doch unter Verwendung von 4-(4-Fluorphenyl)-phenol) | 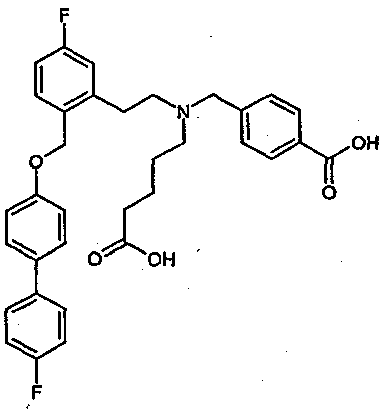 | ¹H-NMR(300MHz):δ [ppm]: (DMSO-d₆): 12,19 (2H, breit), 7,83 (2H, d), 7,67-7,61(2H,m), 7,57 (2H, d), 7,47(1H,dd), 7,35 (2H,d),7,27(2H,dd), 7,10 - 7,02(4H,m),5,03 (2H, s), 3,63 (2H, s), 2,82 (2H, dd), 2,66 (2H, dd), 2,43 (2H),2,11(2H),1,40 (4H). |
| 15 (analog 12, doch unter Verwendung von 4-(4-Methoxy- phenyl)-phenol) | 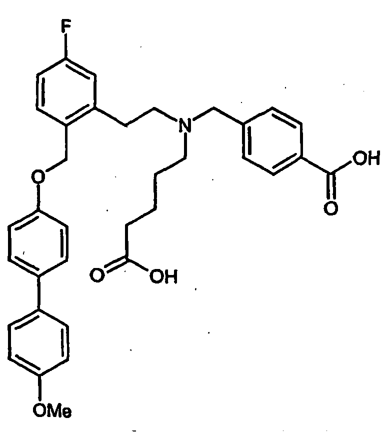 | ¹H-NMR(300MHz):δ [ppm]:(DMSO-d*₆*): 12,49 (2H, breit), 7,83 (2H,d), 7,53 (2H, d), 7,52 (2H, d), 7,47 (1H, dd), 7,33 (2H, d), 7,09-6,97 (6H,m), 5,02 (2H, s), 3,78 (3H,s), 3,63 (2H, s), 2,82 (2H,dd), 2,66 (2H, dd), 2,43 (2H, pseudo-t), 2,11 (2H, pseudo-t), 1,41 (4H). |
| 16 (analog 12, doch unter Verwendung von 4-(4-Chlorphenyl)-phenol) | 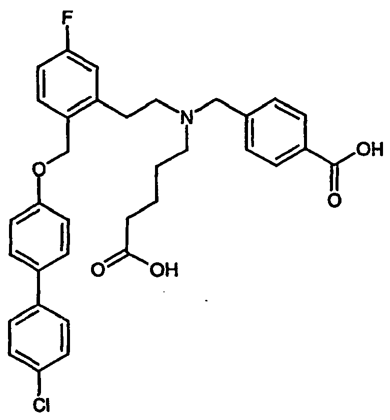 | 12,38 (2H, breit), 7,83 (2H, d), 7,64 (2H, d), 7,60 (2H, d), 7,50-7,47 (3H, m), 7,36 (2H, d), 7,10-7,02 (4H, m), 5,05 (2H, s), 3,63 (2H), 2,82 (2H), 2,66 (2H), 2,43 (2H), 2,10 (2H), 1,41 (4H). 300 (MHz) |
| 17 (analog 12, doch unter Verwendung von 4-(4-Trifluor methylphenyl)- phenol) | 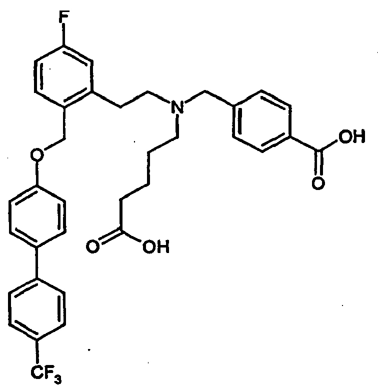 | 12,40 (2H, breit) 7,87-7,74 (6H, m), 7,68 (2H, d), 7,48 (1H, dd), 7,33 (2H, d), 7,10-7,00 (4H, m), 5,07 (2H, s), 3,63 (2H, s), 2,82 (2H, dd), 2,68 (2H, dd), 2,43 (2H), 2,11 (2H), 1,41 (4H). (200 MHz) |
| 18 (analog 12, doch unter Verwendung von 4-(4-Cyano phenyl)-phenol) | 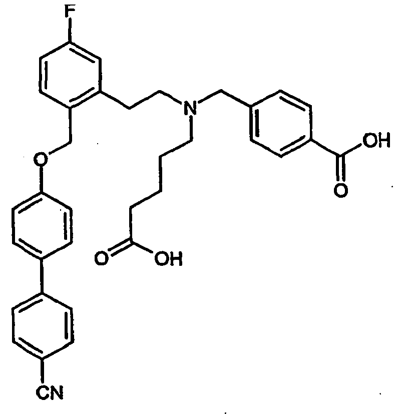 | 12,32 (2H, breit), 7-91-7,80 (6H, m), 7,71 (2H, d), 7,48 (1H, dd), 7,34 (2H, d), 7,09-7,00 (4H, m), 5,07 (2H, s), 3,64 (2H, s), 2,82 (2H), 2,67 (2H), 2,43 (2H), 2,10 (2H), 1,40 (4H). (200 MHz) |
| 19 (analog 12, doch unter Verwendung von 4-(4-Trifluormethylphenyl)-phenol und 2- Brommethylbenzoesäuremethylester) | 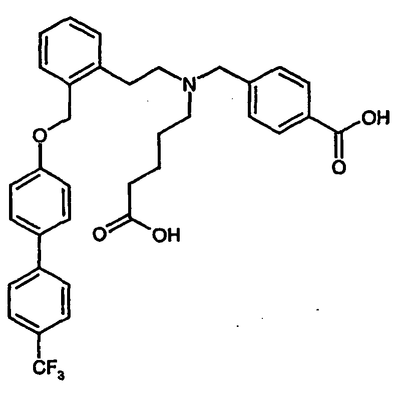 | 12,39 (2H, breit), 7,87-7,74 (6H, m), 7,68 (2H, d), 7,44- 7,33 (3H, m), 7,29-7,17 (3H, m), 7,07 (2H, d), 5,07 (2H, s), 3,66 (2H, s), 2,82 (2H, dd), 2,66 (2H, dd), 2,45 (2H, teilweise überdeckt von DMSO), 2,11 (2H), 1,42 (4H). (200 MHz) |
| 20 (analog 12, doch unter Verwendung von 4-(4-Methoxyphenyl)-phenol und 2-Brommethylbenzoesäuremethylester) | 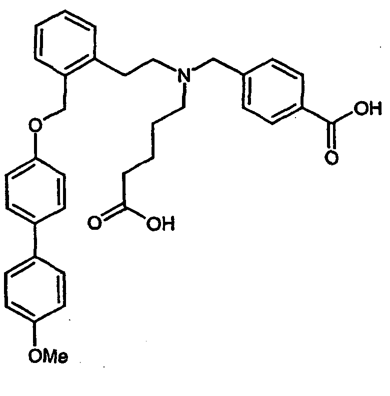 | 12,37 (2H, breit), 7,83 (2H, d), 7,53 (2H, d), 7,52 (2H, d), 7,42-7,37 (3H, m), 7,30- 7,19 (3H, m), 6,98 (4H, d), 5,04 (2H, s), 3,78 (3H, s), 3,65 (2H, s), 2,81 (2H, dd), 2,64 (2H, dd), 2,45 (2H, teilweise überdeckt von DMSO), 2,11 (2H), 1,42 (4H). (300 MHz) |
| 21 (analog 12, doch unter Verwendung von 4-(4-Chlorphenyl)-phenol und 2-Brommethylbenzoesäuremethylester) | 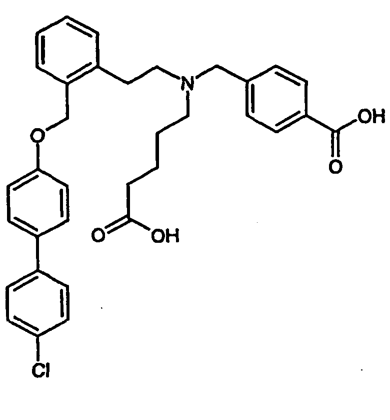 | 12,39 (2H, breit), 7,82 (2H, d), 7,63 (2H, d), 7,59 (2H, d), 7,49-7,34 (5H, m), 7,28- 7,17 (3H, m), 7,02 (2H, d), 5,07 (2H, s), 3,65 (2H, s), 2,80 (2H, dd), 2,63 (2H, dd), 2,45 (2H, teilweise überdeckt von DMSO), 2,11 (2H), 1,42 (4H). (200 MHz) |

## Patentansprüche

1. Verbindungen der allgemeinen Formel (I) 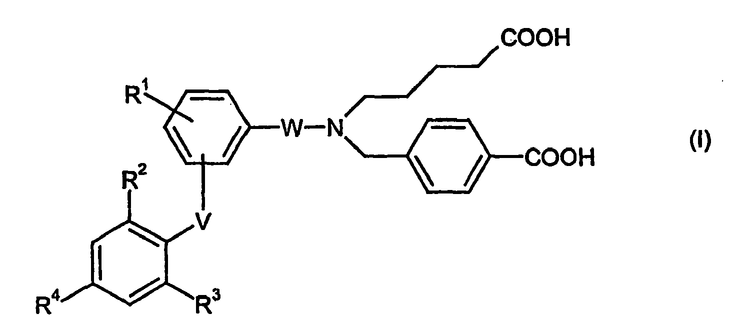 worin
R¹ in meta- oder para-Position zum Rest W angeordnet ist und einen Rest aus der Gruppe, bestehend aus H, Halogen oder OCF₃, bedeutet;
R² H, oder Halogen bedeutet;
R³ H oder Halogen bedeutet;
R⁴ C₁₋₆-Alkyl, C₃₋₈-Cycloalkyl, CF₃, OCF₃, F, Cl, CN, OMe oder Phenyl bedeutet, wobei der Phenylrest zusätzlich einen Substituenten aus der Gruppe, bestehend aus Halogen, CN, C₁₋₆-Alkoxy, CF₃, C₁₋₆-Alkyl, tragen kann;
V in ortho- oder meta-Position zum Rest W angeordnet ist und für O, CH₂O, OCF₂ oder O-C₁₋₆-Alkyl-O steht;
W für CH₂ oder CH₂CH₂ steht; sowie Salze, Isomere und Hydrate davon.

2. Verbindungen nach Anspruch 1, **dadurch, gekennzeichnet, dass**
R¹ in meta-Position zum Rest W angeordnet ist und einen Rest aus der Gruppe, bestehend aus H oder Halogen, bedeutet;
R² H, oder Halogen bedeutet;
R³ H oder Halogen bedeutet;
R⁴ C₁₋₆-Alkyl, C₃₋₈-Cycloalkyl oder Phenyl bedeutet, wobei der Phenylrest zusätzlich einen Substituenten aus der Gruppe, bestehend aus Halogen, CN, C₁₋₆-Alkoxy, CF₃, C₁₋₆-Alkyl, tragen kann;
V in ortho- oder meta-Position zum Rest W angeordnet ist und für O, CH₂O, OCF₂ oder O-C₁₋₆-Alkyl-O steht;
W für CH₂ oder CH₂CH₂ steht;
sowie Salze, Isomere und Hydrate davon.

3. Verbindungen nach Anspruch 1, **dadurch, gekennzeichnet, dass**
R¹ in meta-Position zum Rest W angeordnet ist und einen Rest aus der Gruppe, bestehend aus H, F, Cl oder Br, bedeutet;
R² H bedeutet;
R³ H bedeutet;
R⁴ Methyl, Ethyl, n-Propyl, i-Propyl, n-Butyl, i-Butyl, t-Butyl, Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CN, Methoxy, Ethoxy, n-Propoxy, i-Propoxy, n-Butyloxy, i-Butyloxy, t-Butyloxy, CF₃, Methyl, Ethyl, n-Propyl, i-Propyl, n-Butyl, i-Butyl, t-Butyl, tragen kann;
V in ortho- oder meta-Position zum Rest W angeordnet ist und für O, CH₂O, OCF₂ oder O-C₁₋₆-Alkyl-O steht;
W für CH₂ oder CH₂CH₂ steht;
sowie Salze, Isomere und Hydrate davon.

4. Verbindungen nach Anspruch 1, **dadurch, gekennzeichnet, dass**
R¹ in meta-Position zum Rest W angeordnet ist und H bedeutet;
R² H bedeutet;
R³ H bedeutet;
R⁴ Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CF₃, tragen kann;
V in meta-Position zum Rest W angeordnet ist und für O steht;
W für CH₂ steht;
sowie Salze, Isomere und Hydrate davon.

5. Verbindungen nach Anspruch 1, **dadurch, gekennzeichnet, dass**
R¹ in meta-Position zum Rest W angeordnet ist und H bedeutet;
R² H bedeutet;
R³ H bedeutet;
R⁴ Phenyl bedeutet, wobei der Phenylrest zusätzlich einen Substituenten aus der Gruppe, bestehend aus F, Cl, Br, OMe, CF₃, Methyl, Ethyl, n-Propyl, i-Propyl, n-Butyl, i-Butyl, t-Butyl, tragen kann;
V in ortho-Position zum Rest W angeordnet ist und für OCF₂ steht;
W für CH₂CH₂ steht;
sowie Salze, Isomere und Hydrate davon.

6. Verbindungen nach Anspruch 1, **dadurch, gekennzeichnet, dass**
R¹ in meta-Position zum Rest W angeordnet ist und einen Rest aus der Gruppe, bestehend aus H, F, Cl oder Br, bedeutet;
R² H bedeutet;
R³ H bedeutet;
R⁴ Methyl, Ethyl, n-Propyl, i-Propyl, n-Butyl, i-Butyl, t-Butyl, Cyclopropyl, Cyclobutyl, Cyclopentyl, Cyclohexyl, oder Phenyl bedeutet, wobei der Phenylrest zusätzlich einen Substituenten aus der Gruppe, bestehend aus F, Cl, Br, CN, OMe, CF₃, Methyl, Ethyl, n-Propyl, i-Propyl, n-Butyl, i-Butyl, t-Butyl, tragen kann;
V in ortho-Position zum Rest W angeordnet ist und für CH₂O steht;
W für CH₂CH₂ steht;
sowie Salze, Isomere und Hydrate davon.

7. Verfahren zur Herstellung von Verbindungen der allgemeinen Formel (I), **dadurch gekennzeichnet, dass** man
Verbindungen der Formel (II) 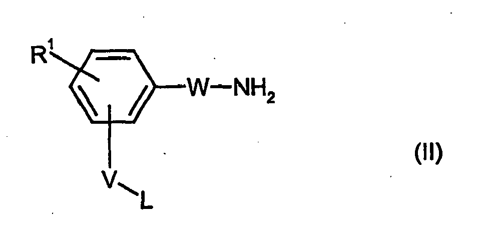 worin
R¹, V und W die in Anspruch 1 angegebenen Bedeutungen haben, und
L für den Fall, dass V gleich O ist, für Methyl oder ansonsten für einen Rest der Formel 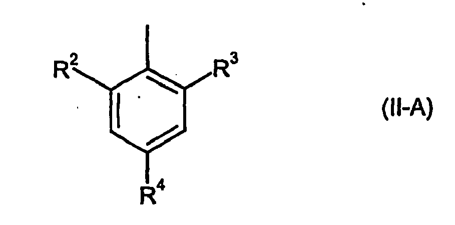 steht, wobei R², R³ und R⁴ die in Anspruch 1 angegebenen Bedeutungen haben,
mit einem 4-Formylbenzoesäure-C₁₋₆-alkylester in einem organischen Lösungsmittel gegebenenfalls unter Erhitzen und gleichzeitiger oder anschließender Zugabe eines Reduktionsmittels zu Verbindungen der Formel (III) umsetzt, 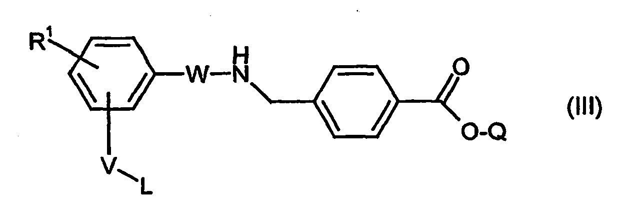 worin R¹, V, W und L die vorstehend angegebenen Bedeutungen haben und Q für einen C₁₋₆-Alkylrest steht,
anschließend - gegebenenfalls unter vorheriger Etherspaltung zur freien Hydroxylgruppe, wenn V für O und L für Methyl steht - mit einem ω-Halogenvaleriansäure-C₁₋₆-alkylester in einem organischen Lösungsmittel in Gegenwart einer Base unter Erhitzen zu Verbindungen der Formel (IV) umsetzt, 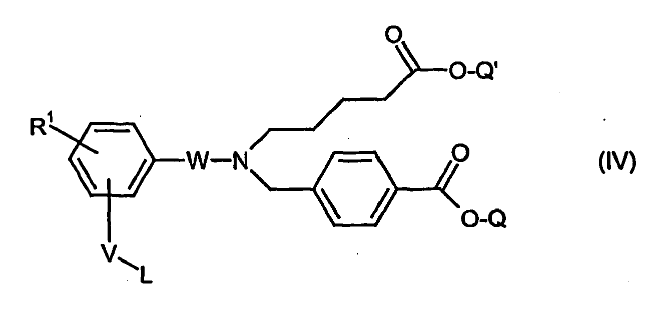 worin R¹, V,W und Q die vorstehend angegebenen Bedeutungen haben, Q' für einen C₁₋₆-Alkylrest steht und L für H - wenn V gleich O ist - oder einen Rest der Formel II-A steht,
anschließend - für den Fall, dass V gleich O und L für H steht - mit einer Verbindung der Formel IV-A in einem organischen Lösungsmittel unter Erhitzen 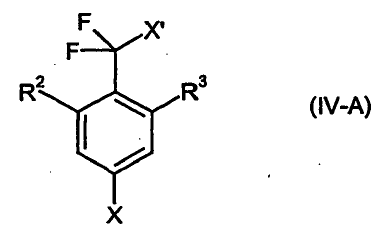 wobei R² und R³ die in Anspruch 1 angegebene Bedeutung haben und X und X' jeweils für Halogen stehen,
und anschließender Palladium-katalysierter Substitution des Restes X mit einem Benzolboronsäurederivat zu Verbindungen der Formel (V) umsetzt, 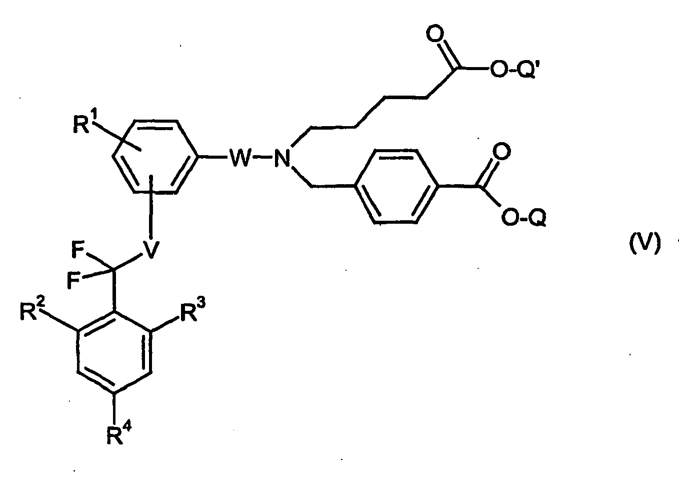 und anschließend die Verbindungen der Formel (IV) beziehungsweise (V) unter alkalischen Bedingungen zu den Verbindungen der Formel (I) hydrolysiert.

8. Verbindungen nach einem der Ansprüche 1 bis 6 zur Behandlung von Krankheiten.

9. Arzneimittel enthaltend mindestens eine Verbindung der allgemeinen Formel (I) gemäß einem der vorhergehenden Ansprüche.

10. Verwendung von Verbindungen der Formel (I) gemäß einem der Ansprüche 1 bis 8 zur Herstellung eines Arzneimittels zur Behandlung von Herz-Kreislauf-Erkrankungen.

11. Verwendung von Verbindungen der allgemeinen Formel (I) gemäß einem der Ansprüche 1 bis 8 zur Herstellung von Arzneimitteln zur Behandlung von Angina pectoris, Ischämien und Herzinsuffizienz.

12. Verwendung von Verbindungen der allgemeinen Formel (I) gemäß einem der Ansprüche 1 bis 8 zur Herstellung von Arzneimitteln zur Behandlung von Hypertonie, thromboembolischen Erkrankungen, Arteriosklerose und venösen Erkrankungen.

13. Verwendung von Verbindungen der allgemeinen Formel (I) gemäß einem der Ansprüche 1 bis 8 zur Herstellung von Arzneimitteln zur Behandlung von fibrotischen Erkrankungen.

14. Verwendung nach Anspruch 13, **dadurch gekennzeichnet, dass** die fibrotische Erkrankung Leberfibrose ist.

## Claims

1. Compounds of the general formula (I) 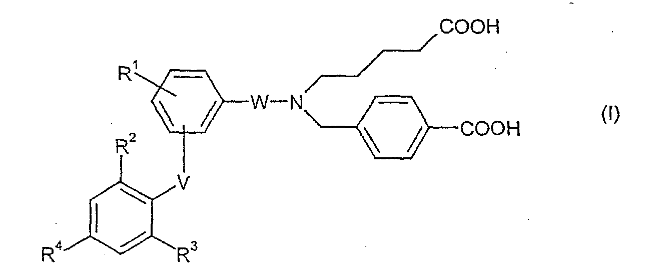 in which
R¹ is located in the meta- or para-position to the radical W and represents a radical from the group consisting of H, halogen and OCF₃;
R² represents H, or halogen;
R³ represents H or halogen;
R⁴ represents C₁₋₆-alkyl, C₃₋₈-cycloalkyl, CF₃, OCF₃, F, Cl, CN, OMe or phenyl, where the phenyl radical may additionally carry a substituent from the group consisting of halogen, CN, C₁₋₆-alkoxy, CF₃, C₁₋₆-alkyl;
V is located in the ortho- or meta-position to the radical W and represents O, CH₂O, OCF₂ or O-C₁₋₆-alkyl-O;
W represents CH₂ or CH₂CH₂;
and salts, isomers and hydrates thereof.

2. Compounds according to Claim 1, **characterized in that**
R¹ is located in the meta-position to the radical W and represents a radical from the group consisting of H and halogen;
R² represents H, or halogen;
R³ represents H or halogen;
R⁴ represents C₁₋₆-alkyl, C₃₋₈-cycloalkyl or phenyl, where the phenyl radical may additionally carry a substituent from the group consisting of halogen, CN, C₁₋₆-alkoxy, CF₃, C₁₋₆-alkyl;
V is located in the ortho- or meta-position to the radical W and represents O, CH₂O OCF₂ or O-C₁₋₆-alkyl-O;
W represents CH₂ or CH₂CH₂;
and salts, isomers and hydrates thereof.

3. Compounds according to Claim 1, **characterized in that**
R¹ is located in the meta-position to the radical W and represents a radical from the group consisting of H, F, Cl and Br;
R² represents H,
R³ represents H;
R⁴ represents methyl, ethyl, n-propyl, i-propyl, n-butyl, i-butyl, t-butyl, cyclopropyl, cyclobutyl, cyclopentyl, cyclohexyl, or phenyl, where the phenyl radical may additionally carry a substituent from the group consisting of F, Cl, Br, CN, methoxy, ethoxy, n-propoxy, i-propoxy, n-butyloxy, i-butyloxy, t-butyloxy, CF₃, methyl, ethyl, n-propyl, i-propyl, n-butyl, i-butyl, t-butyl;
V is located in the ortho- or meta-position to the radical W and represents 0, CH₂O, OCF₂ or O-C₁₋₆-alkyl-O;
W represents CH₂ or CH₂CH₂;
and salts, isomers and hydrates thereof.

4. Compounds according to Claim 1, **characterized in that**
R¹ is located in the meta-position to the radical W and represents H;
R² represents H;
R³ represents H;
R⁴ represents cyclopropyl, cyclobutyl, cyclopentyl, cyclohexyl, or phenyl, where the phenyl radical may additionally carry a substituent from the group consisting of F, Cl, Br, CF₃;
V is located in the meta-position to the radical W and represents O;
W represents CH₂;
and salts, isomers and hydrates thereof.

5. Compounds according to Claim 1, **characterized in that**
R¹ is located in the meta-position to the radical W and represents H;
R² represents H;
R³ represents H;
R⁴ represents phenyl, where the phenyl radical may additionally carry a substituent from the group consisting of F, Cl, Br, OMe, CF₃, methyl, ethyl, n-propyl, i-propyl, n-butyl, i-butyl, t-butyl;
V is located in the ortho-position to the radical W and represents OCF₂;
W represents CH₂CH₂;
and salts, isomers and hydrates thereof.

6. Compounds according to Claim 1, **characterized in that**
R¹ is located in the meta-position to the radical W and represents a radical from the group consisting of H, F, Cl and Br;
R² represents H;
R³ represents H;
R⁴ represents methyl, ethyl, n-propyl, i-propyl, n-butyl, i-butyl, t-butyl, cyclopropyl, cyclobutyl, cyclopentyl, cyclohexyl, or phenyl, where the phenyl radical may additionally carry a substituent from the group consisting of F, Cl, Br, CN, OMe, CF₃, methyl, ethyl, n-propyl, i-propyl, n-butyl, i-butyl, t-butyl;
V is located in the ortho-position to the radical W and represents CH₂O;
W represents CH₂CH₂;
and salts, isomers and hydrates thereof.

7. Process for preparing compounds of the general formula (I), **characterized in that** compounds of the formula (II) 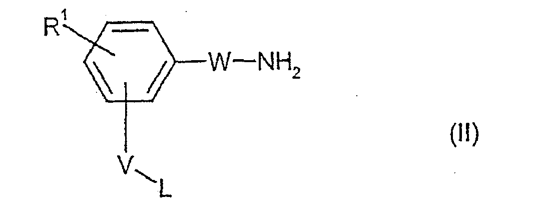 in which
R¹, V and W are as defined in Claim 1 and
L, if V is O, represents methyl or otherwise represents a radical of the formula 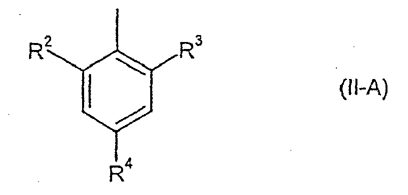
where R², R³ and R⁴ are as defined in Claim 1,
are reacted with a C₁₋₆-alkyl 4-formylbenzoate in an organic solvent, if appropriate with heating and simultaneous or subsequent addition of a reducing agent, to give compounds of the formula (III) 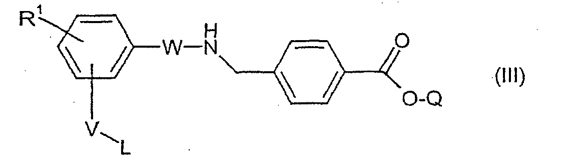 in which R¹, V, W and L are as defined above and Q represents a C₁₋₆-alkyl radical,
then - if appropriate with prior cleavage of the ether to give the free hydroxyl group, if V represents O and L represents methyl - reacted with a C₁₋₆-alkyl ω-halovalerate in an organic solvent in the presence of a base with heating to give compounds of the formula (IV) 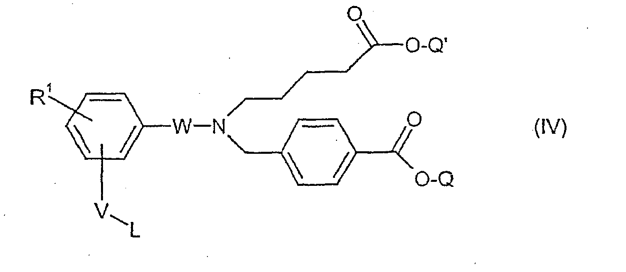 in which R¹, V, W, and Q are as defined above, Q' represents a C₁₋₆-alkyl radical and L represents H - if V is O - or a radical of the formula II-A,
then - if V is O and L represents H - reacted with a compound of the formula IV-A in an organic solvent with heating 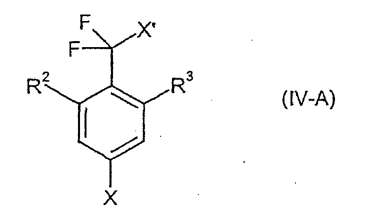 where R² and R³ are as defined in Claim 1 and X and X' each represent halogen,
followed by palladium-catalyzed substitution of the radical X with a benzene boronic acid derivative to give compounds of the formula (V) 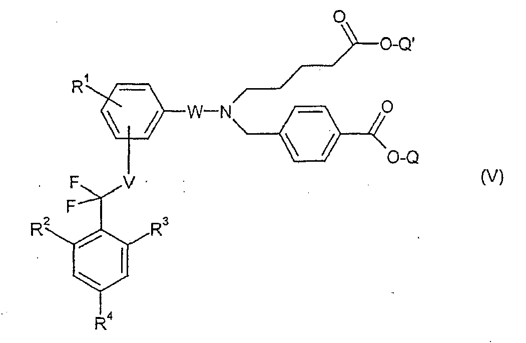 and subsequent hydrolysis of the compounds of the formula (IV) or (V) under alkaline conditions to give the compounds of the formula (I).

8. Compounds according to any of Claims 1 to 6 for treating diseases.

9. Medicament, comprising at least one compound of the general formula (I) according to any of the preceding claims.

10. Use of compounds of the formula (I) according to any of Claims 1 to 8 for preparing a medicament for treating cardiovascular disorders.

11. Use of compounds of the general formula (I) according to any of Claims 1 to 8 for preparing medicaments for treating angina pectoris, ischemias and heart failure.

12. Use of compounds of the general formula (I) according to any of Claims 1 to 8 for preparing medicaments for treating hypertension, thromboembolic disorders, arteriosclerosis and venous diseases.

13. Use of compounds of the general formula (I) according to any of Claims 1 to 8 for preparing medicaments for treating fibrotic disorders.

14. Use according to Claim 13, **characterized in that** the fibrotic disorder is fibrosis of the liver.

## Revendications

1. Composés de formule générale (I) 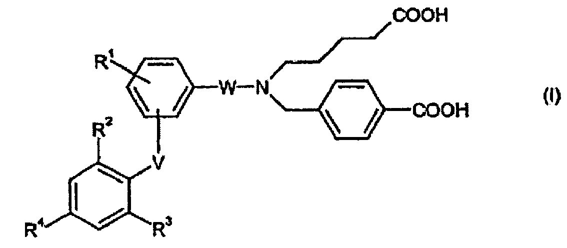 dans laquelle
R¹ se trouve en position méta ou para par rapport au reste W et représente un reste du groupe constitué de H, halogène ou OCF₃;
R² représente H ou un halogène;
R³ représente H ou un halogène;
R⁴ est un reste alkyle en C₁ à C₆, cycloalkyle en C₃ à C₈, CF₃, OCF₃, F, Cl, CN, OMe ou phényle, le reste phényle pouvant porter en outre un substituant du groupe constitué d'halogène, CN, alkoxy en C₁ à C₆, CF₃, alkyle en C₁ à C₆;
V se trouve en position ortho ou méta par rapport au reste W et représente O, CH₂O, OCF₂ ou O-(alkyle en C₁ à C₆)-O;
W représente un reste CH₂ ou CH₂CH₂;
ainsi que leurs sels, leurs isomères et leurs hydrates.

2. Composés suivant la revendication 1, **caractérisés en ce que**
R¹ se trouve en position méta par rapport au reste W et représente un reste du groupe constitué de H ou un halogène,
R² représente H ou un halogène,
R³ représente H ou un halogène,
R⁴ est un reste alkyle en C₁ à C₆, cycloalkyle en C₃ à C₈ ou phényle, le reste phényle pouvant porter en outre un substituant du groupe constitué d'halogène, CN, alkoxy en C₁ à C₆, CF₃, alkyle en C₁ à C₆;
W représente CH₂ ou CH₂CH₂;
ainsi que leurs sels, leurs isomères et leurs hydrates.

3. Composés suivant la revendication 1, **caractérisés en ce que**
R¹ se trouve en position méta par rapport au reste W et représente un reste du groupe constitué de H, F, Cl ou Br;
R² représente H;
R³ représente H;
R⁴ représente un reste méthyle, éthyle, n-propyle, isopropyle, n-butyle, isobutyle, tertio-butyle, cyclopropyle, cyclobutyle, cyclopentyle, cyclohexyle ou phényle, le reste phényle pouvant en outre porter un substituant du groupe constitué de F, Cl, Br, CN, méthoxy, éthoxy, n-propoxy, isopropoxy, n-butyloxy, isobutyloxy, tertio-butyloxy, CF₃, méthyle, éthyle, n-propyle, isopropyle, n-butyle, isobutyle, tertiobutyle;
V se trouve en position ortho ou méta par rapport au reste W et représente O, CH₂O OCF₂ ou O-(alkyle en C₁ à C₆)-O;
W représente un reste CH₂ ou CH₂CH_{2;}
ainsi que leurs sels, leurs isomères et leurs hydrates.

4. Composés suivant la revendication 1, **caractérisés en ce que**
R¹ se trouve en position méta par rapport au reste W et représente H;
R² représente H;
R³ représente H;
R⁴ est un reste cyclopropyle, cyclobutyle, cyclopentyle, cyclohexyle ou phényle, le reste phényle pouvant en outre porter un substituant du groupe constitué de F, Cl, Br, CF₃;
V se trouve en position méta par rapport au reste W et représente O;
W représente CH₂;
ainsi que leurs sels, leurs isomères et leurs hydrates.

5. Composés suivant la revendication 1, **caractérisés en ce que**
R¹ se trouve en position méta par rapport au reste W et représente H;
R² représente H;
R³ représente H;
R⁴ représente un reste phényle, le reste phényle pouvant en outre porter un substituant du groupe constitué de F, Cl, Br, OMe, CF₃, méthyle, éthyle, n-propyle, isopropyle, n-butyle, isobutyle, tertio-butyle;
V se trouve en position ortho par rapport au reste W et représente OCF₂;
W est un reste CH₂CH₂,
ainsi que leurs sels, leurs isomères et leurs hydrates.

6. Composés suivant la revendication 1, **caractérisés en ce que**
R¹ se trouve en position méta par rapport au reste W et représente un reste du groupe constitué de H, F, Cl ou Br;
R² représente H;
R³ représente H;
R⁴ est un reste méthyle, éthyle, n-propyle, isopropyle, n-butyle, isobutyle, tertio-butyle, cyclopropyle, cyclobutyle, cyclopentyle, cyclohexyle ou phényle, le reste phényle pouvant en outre porter un substituant du groupe constitué de F, Cl, Br, CN, OMe, CF₃, méthyle, éthyle, n-propyle, isopropyle, n-butyle, isobutyle, tertio-butyle;
V se trouve en position ortho par rapport au reste W et représente CH₂O;
W représente CH₂CH₂;
ainsi que leurs sels, leurs isomères et leurs hydrates.

7. Procédé de production de composés de formule générale (I), **caractérisé en ce que :**
on fait réagir des composés de formule (II) 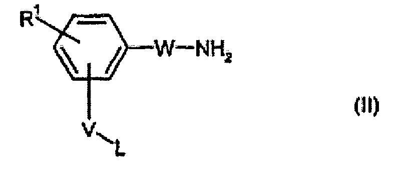
dans laquelle
R¹, V et W ont les définitions indiquées dans la revendication 1, et
L est un reste méthyle, au cas où V représente O, sinon il est un reste de formule 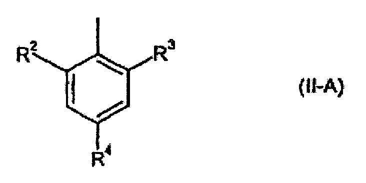
dans laquelle
R², R³ et R⁴ ont les définitions indiquées dans la revendication 1,
avec un ester d'alkyle en C₁ à C₆ d'acide 4-formylbenzoïque dans un solvant organique, le cas échéant avec chauffage et addition simultanée ou subséquente d'un agent réducteur, pour obtenir des composés de formule (III) 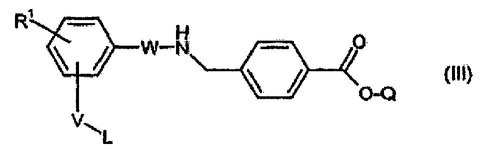 dans laquelle R¹, V, W et L ont les définitions indiquées ci-dessus et Q représente un reste alkyle en C₁ à C₆, qu'on fait ensuite réagir - éventuellement avec coupure préalable de l'éther en groupe hydroxyle libre, lorsque V représente O et L est un groupe méthyle - avec un ester d'alkyle en C₁ à C₆ d'acide ω-halogénovalérique dans un solvant organique en chauffant en présence d'une base pour former des composés de formule (IV) 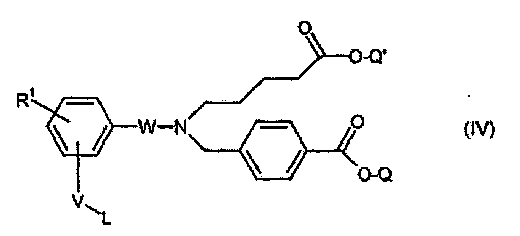 dans laquelle R¹, V, W et Q ont les définitions indiquées ci-dessus, Q' est un reste alkyle en C₁ à C₆ et L représente H - lorsque V représente O - ou un reste de formule II-A,
qu'on fait réagir ensuite - au cas où V représente O et L représente H - dans un solvant organique, en chauffant, avec un composé de formule IV-A 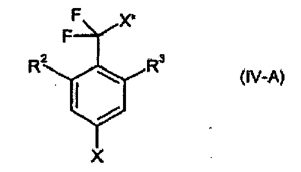 dans laquelle R² et R³ ont la définition indiquée dans la revendication 1 et X et X' représentent chacun un halogène,
avec ensuite substitution, catalysée au palladium, du reste X avec un dérivé d'acide benzène-boronique pour obtenir des composés de formule (V) 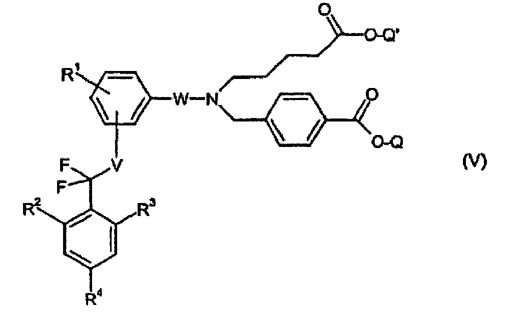 puis on hydrolyse les composés de formule (IV) ou (V) dans des conditions alcalines pour obtenir les composés de formule (I).

8. Composés suivant l'une des revendications 1 à 6, destinés au traitement de maladies.

9. Médicament contenant au moins un composé de formule générale (I) suivant l'une des revendications précédentes.

10. Utilisation de composés de formule (I) suivant l'une des revendications précédentes pour la préparation d'un médicament destiné au traitement de maladies du système cardiovasculaire.

11. Utilisation de composés de formule générale (I) suivant l'une des revendications précédentes pour la préparation de médicaments destinés au traitement de l'angine de poitrine, d'ischémies et de l'insuffisance cardiaque.

12. Utilisation de composés de formule générale (I) suivant l'une des revendications précédentes pour la préparation de médicaments destinés au traitement de l'hypertonie, de maladies thromboemboliques, de l'artériosclérose et d'affections veineuses.

13. Utilisation de composés de formule générale (I) suivant l'une des revendications précédentes pour la préparation de médicaments destinés au traitement de fibroses.

14. Utilisation suivant la revendication 12, **caraactérisée en ce que** la fibrose est la fibrose hépatique.
